(19) Europäisches Patentamt
European Patent Office
Office européen des brevets

(11) **EP 0 972 016 B1**

(12) **EUROPEAN PATENT SPECIFICATION**

(45) Date of publication and mention
of the grant of the patent:
**31.03.2010 Bulletin 2010/13**

(21) Application number: **98900849.5**

(22) Date of filing: **30.01.1998**

(51) Int Cl.:
*C12N 9/42* (2006.01)

(86) International application number:
**PCT/DK1998/000039**

(87) International publication number:
**WO 1998/033895 (06.08.1998 Gazette 1998/31)**

(54) **A THERMOSTABLE ENDO-BETA-1,4-GLUCANASE**

THERMOSTABILE ENDO-BETA-1,4-GLUCANASE

ENDO-BETA-1,4-GLUCANASE THERMOSTABLE

(84) Designated Contracting States:
**AT BE CH DE DK ES FI FR GB GR IE IT LI NL PT SE**

(30) Priority: **31.01.1997 DK 11497**
**11.07.1997 DK 85397**

(43) Date of publication of application:
**19.01.2000 Bulletin 2000/03**

(83) **Declaration under Rule 32(1) EPC (expert solution)**

(73) Proprietor: **Novozymes A/S**
**2880 Bagsvaerd (DK)**

(72) Inventors:
• **ANDERSEN, Lene, Nonboe**
**DK-3450 Allerod (DK)**
• **BJORNVAD, Mads, Eskelund**
**DK-1955 Frederiksberg (DK)**
• **SCHÜLEIN, Martin**
**DK-2100 Copenhagen (DK)**

(56) References cited:
**US-A- 5 275 944**

• **DIALOG INFORMATION SERVICES, File 5, BIOSIS PREVIEWS, Dialog Accession No. 10020697, Biosis No. 95020697, TIKHOMIROV D.F. et al., "Highly Thermostable Cellulase Complex of an Extremely Thermophilic Bacterium Anaerocellum-Thermophilum"; & PRIKL. BIOKHIM. MIKROBIOL., 28(3), 1992, 339-347.**
• **CHEMICAL ABSTRACTS, Volume 95, No. 17, 26 October 1981, (Columbus, Ohio, USA), PETRE JEAN et al., "Purification and Properties of an Endo-Beta-1,4-Glucanase from Clostridium Thermocellum", Abstract No. 145879q; & BIOCHIMIE, 1981, 63(7), 629-639.**
• **DIALOG INFORMATION SERVICES, File 5, Biosis, Dialog Accession No. 659397, Biosis Accession No. 90027397, BRONNENMEIER K. et al., "Cellulose Hydrolysis by a Highly Thermostable Endo-1 4-beta Glucanase Vicelase I from Clostridium-Stercorarium"; & ENZYME MICROB. TECHNOL., 12(6), 1990, 431-436.**
• **CHEMICAL ABSTRACTS, Volume 111, No. 23, 4 December 1989, (Columbus, Ohio, USA), BRAGGER J.M. et al., "Very Stable Enzymes from Extremely Thermophilic Archaebacteria and Eubacteria", page 317, Abstract No. 211647q; & MICROBIOL. BIOTECHNOL., 1989, 31(5-6), 556-561.**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF INVENTION**

The present invention relates to an enzyme with cellulolytic activity at high temperature, especially an endo-beta-1,4-glucanase; a cloned DNA sequence encoding the enzyme with cellulolytic activity; a method for providing a gene encoding such an enzyme; a method of producing the enzyme; an enzyme composition comprising the enzyme with cellulolytic activity; and the use of said enzyme and enzyme composition for a number of industrial applications.

**BACKGROUND OF THE INVENTION**

Cellulases or cellulolytic enzymes are enzymes involved in hydrolysis of cellulose. In the hydrolysis of native cellulose, it is known that there are three major types of cellulase enzymes involved, namely cellobiohydrolase (1,4-beta-D-glucan cellobiohydrolase, EC 3.2.1.91), endo-beta-1,4-glucanase (endo-1,4-neta-D-glucan 4-glucanohydrolase, EC 3.2.1.4) and beta-glucosidase (EC 3.2.1.21).

Especially the endo-beta-1,4-glucanases (EC No. 3.2.1.4) constitute an interesting group of hydrolases for the mentioned industrial uses. Endo-beta-1,4-glucanases catalyses endo hydrolysis of 1,4-beta-D-glycosidic linkages in cellulose, cellulose derivatives (such as carboxy methyl cellulose and hydroxy ethyl cellulose), lichenin, beta-1,4 bonds in mixed beta-1,3 glucans such as cereal beta-D-glucans or xyloglucans and other plant material containing cellulosic parts. The authorized name is endo-1,4-beta-D-glucan 4-glucano hydrolase, but the abbreviated term endo-beta-1,4-glucanase is used in the present specification. Reference can be made to T.-M. Enveri, "Microbial Cellulases" in W.M. Fogarty, Microbial Enzymes and Biotechnology, Applied Science Publishers, p. 183-224 (1983); Methods in Enzymology, (1988) Vol. 160, p. 200-391 (edited by Wood, W.A. and Kellogg, S.T.); Béguin, P., "Molecular Biology of Cellulose Degradation", Annu. Rev. Microbiol. (1990), Vol. 44, pp. 219-248; Béguin, P. and Aubert, J-P., "The biological degradation of cellulose", FEMS Microbiology Reviews 13 (1994) p.25-58; Henrissat, B., "Cellulases and their interaction with cellulose", Cellulose (1994), Vol. 1, pp. 169-196.

Cellulases are synthesized by a large number of microorganisms which include fungi, actinomycetes, myxobacteria and true bacteria but also by plants. Especially endo-beta-1,4-glucanases of a wide variety of specificities have been identified. Many bacterial endo-beta-1,4-glucanases have been described (Henrissat, B. and Bairoch, A. (1993) Biochem J. 293:781-788; Gilbert, H.J. and Hazlewood, G.P. (1993) J. Gen. Microbiol. 139:187-194).

The superior taxonomic group Archaea is divided in two divisions Euryarchaeota and Crenarchaeota. The *Thermococcales* group in the Euryarchaeota division of Archaea comprises the genera *Pyrococcus* and *Thermococcus* (Maidak et al. (1996)). These organisms are isolated from marine or terrestrial sulfataric situations, and are anaerobic, hyper-thermophiles. *Pyrococcus furiosus* shows optimal growth at 100°C, and maximum temperature of growth is at 103°C. Optimum growth is obtained in the presence of 15-35 g/l of NaCl. As carbon sources *P. furiosus* is described to utilize peptides, proteins, starch as well as maltose (Zillig (1992)).

Various extracellular enzymes have been described from Archaeon species. For example, from *P. furiosus,* DSM 3638, an amylase with temperature optimum at approx. 100°C has been cloned and expressed in *E. coli* and *B. subtilis* (International patent application PCT/DK/90/00074). Also other hydrolytic enzymes such as extracellular xylanases, beta-mannosidases and beta-galactosidases and an intracellular protease have been described from various archaeon species (Halio et al. (1996)).

A beta-glucosidase has been described, characterized and cloned from *Pyrococcus furiosus,* DSM 3638, (Kengen (1993)). The characterization of the *P. furiosus* beta-glucosidase revealed that this enzyme has no activity on cellulose or other beta-linked glucose polymers (Bauer et al. (1996)). In WO 97/44361 (page 115-117) is disclosed a polynuclotide sequence encoding a polypeptide having endo-beta-1,4-glucanase activity which is derived from *Pyrococcus furiosus. Thermophilum pendens,* a species related to *Pyrobaculum* in the Crenarchaeota division of Archaea has been shown to produce small amounts of a glucanase with activity against carboxymethyl cellulose at 88°C. However, no further characterization of neither enzyme or isolates is described (Bragger et al. (1989)).

Xyloglucanase activity is not included in the classification of enzymes provided by the Enzyme Nomenclature (1992). Hitherto, this enzymatic activity has simply been classified as glucanase activity and has often been believed to be identical to cellulolytic activity (EC 3.2.1.4), i.e. activity against β-1,4-glycosidic linkages in cellulose or cellulose derivative substrates, or at least to be a side activity in enzymes having cellulolytic activity. However, a true xyloglucanase is a true xyloglucan specific enzyme capable of catalyzing the solubilisation of xyloglucan to xyloglucan oligosaccharides but which does not exhibit substantial cellulolytic activity, e.g. activity against the conventionally used cellulose-like substrates CMC (carboxymethylcellulose), HE cellulose and Avicel (microcrystalline cellulose). A xyloglucanase cleaves the beta-1,4-glycosidic linkages in the backbone of xyloglucan.

Xyloglucanase activity is described by Vincken et al. (1997) who characterizes three different endo-beta-1,4-glucanases from Trichoderma viride (similar to T. reesei) which all have high activity against cellulose or CMC and show that the EndoI (which is indeed belonging to family 5 of glycosyl hydrolases, see Henrissat, B. et al. (1991, 1993)) has essentially no (i.e. very little) activity against xyloglucan, and that EndoV (belonging to the family 7 of glycosyl hydrolases) and EndoIV (belonging to the family 12 of glycosyl hydrolases) both have activity against xyloglucan and CMC, respectively, of the same order of magnitude. Accordingly, it is known that family 12 endo-beta-1,4-glucanases may also exhibit xyloglucanase activity.

A very important industrial use of cellulolytic enzymes is the use for treatment of cellulosic fibers, textile or fabric, e.g. as ingredients in detergent compositions or fabric softener compositions, for enzymatic scouring of cellulosic material for the textile industry, for desizing of woven fabric, for biopolishing of new fabric (garment finishing), and for obtaining a "stone-washed" look of cellulose-containing fabric, especially denim, and several methods for such treatment have been suggested, e.g. in GB-A-1 368 599, EP-A-0 307 564 and EP-A-0 435 876, WO 91/17243, WO 91/10732, WO 91/17244, PCT/DK95/000108 and PCT/DK95/00132. Another important industrial use of cellulytic enzymes is the use for treatment of paper pulp, e.g. for improving the drainage or for deinking of recycled paper.

It is also known that cellulases may or may not have a cellulose binding domain (a CBD). The CBD enhances the binding of the enzyme to a cellulose-containing fiber and increases the efficacy of the catalytic active part of the enzyme.

There is an ever existing need for providing novel cellulase enzyme preparations which may be used for applications where cellulase, preferably an endo-beta-1,4-glucanase, activity is desirable.

The object of the present invention is to provide novel enzyme compositions having substantial cellulytic activity at high temperature conditions and improved performance in paper pulp processing, textile treatment, laundry processes, extraction processes or in animal feed; preferably novel cellulases, more preferably well-performing endo-beta-1,4-glucanases, which are contemplated to be producible or produced by recombinant techniques.

## SUMMARY OF THE INVENTION

The inventors have now succeeded in cloning and characterizing a DNA sequence from the archaeal species *Pyrococcus furiosus* which encodes an enzyme exhibiting cellulolytic activity at extremely high temperature in a very broad pH range, thereby making it possible to prepare a monocomponent cellulolytic enzyme composition with desired properties.

Accordingly, in a first aspect the invention relates to an enzyme having endo-β-1,4-glucanase activity which has optimum activity at a temperature of at least 90 °C, preferably af at least 95°C, more preferably of at least 100°C.

In its second aspect, the invention relates to a DNA construct comprising a DNA sequence encoding an enzyme halving endo-beta-1,4-glucanase activity and optimum activity above 90°C, preferably above 95°C, more preferably above 100°C, which DNA sequence comprises

a) the DNA sequence corresponding to the endo-beta-1,4-glucanase encoding part of the DNA sequence obtainable from the plasmid in *Escherichia coli* DSM 11203, or

b) an analogue of the DNA sequence corresponding to the endo-beta-1,4-glucanase encoding part of the DNA sequence of SEQ ID NO:1 or the DNA sequence obtainable from the plasmid in *Escherichia coli* DSM 11203, which

i) is homologous, preferably at least 80% identical, with the DNA sequence corresponding to the endo-beta-1,4-glucanase encoding part of the DNA sequence obtainable from the plasmid in *Escherichia coli* DSM 11203, or

ii) encodes a polypeptide which is at least 50% identical with the polypeptide of SEQ ID NO: 2, or the polypeptide encoded by a DNA sequence comprising the DNA sequence corresponding to the endo-beta-1,4-glucanase encoding part of the DNA sequence obtainable from the plasmid in *Escherichia coli* DSM 11203, or

In its third, fourth and fifth aspect the invention provides an expression vector harbouring the cloned DNA sequence of the invention, a cell comprising the expression vector and a method of producing an enzyme exhibiting cellulolytic activity, which method comprises culturing the cell under conditions permitting the production of the enzyme, and recovering the enzyme from the culture.

In yet another aspect the invention provides an isolated enzyme exhibiting cellulolytic activity, characterized in (i) being free from homologous impurities and (ii) the enzyme is produced by the method described above.

The invention further relates to an isolated enzyme having cellulolytic activity, preferably an endo-beta-1,4-glucanase, which is encoded by the DNA construct of the invention. Further, the present invention relates to the use of such an enzyme or the enzyme preparation of the invention for industrial applications such is in the textile industry for improving the properties of cellulosic fibres or fabric or for providing a stone-washed look of denim; or in the cellulosic fibre processing industy, especially for the retting of hemp, jute, flax or linen; or in industrial cleaning processed; or in heat extruded polymeric material; or in the conversion of biomass to sugars; or in the production of alcohol; or for predigestion of e.g. grains used in the feed production; or in the production of instant coffee or similar extraction processes; or in the the oil-drilling industry; or in enhanced oil recovery by polymer flooding.

The invention also relates to an isolated substantially pure biological culture of the *Escherichia coli* strain DSM 11203 harbouring a cellulase, encoding DNA sequence (the cellulase encoding part of the DNA sequence clones into plasmid pSJ1678 present in *Escherichia coli* ISM 11203 derived from a strain of the archaeal species *Pyrococcus furiosus,* or any mutant of said *E. coli* strain).

## DETAILED DESCRIPTION OF THE INVENTION

In the present context, the term "the 20 naturally occuring amino acid residues" denotes the 20 amino acid residues usually found in proteins and conventionally known as alanine (Ala or A), valine (Val or V), leucine (Leu or L), isoleucine (Ile or I), proline (Pro or P), phenylalanine (Phe or F), tryptophan (Trp or W), methionine (Met or M), glycine (Gly or G), serine (Ser or S), threonine (Thr or T), cysteine (Cys or C), tyrosine (Tyr or Y), asparagine (Asn or N), glutamine (Gln or Q), aspartic acid (Asp or D), glutamic acid (Glu or E), lysine (Lys or K), arginine (Arg or R), and histidine (His or H).

The enzyme and the enzyme preparation of the invention is active over a broad pH range, preferably active at a pH between about 4 and about 11, preferably between about 5.5 and about 10.

In a preferred embodiment, the enzyme or the enzyme preparation of the invention is obtainable from or endogeneous to a strain belonging to Archaea, more preferably to a strain belonging to the division Euryarchaeota, even more preferably belonging to the genus *Pyrococcus*, especially being the species *Pyrococcus furiosus,* such as *Pyrococcus furiosus,* DSM 3638. Further, it is believed that the enzyme of the invention is covered by the definition of family 12 glycosyl hydrolases, i.e. the enzyme of the present invention preferably is a family 12 endo-β-1,4-glucanase. The enzyme of the invention may have a molecular weight of about 34 kDa. However, it is believed that the enzyme of the invention may also have an apparent molecular weight from about 30 kDa to about 35 kDa (i.e. 30, 31, 32, 33, 34 ot 35 kDa) depending on possible proteolytic activity on the N-terminal amino acid residue sequence of the enzyme during the fermentation and/or purification processes. In other words, the enzyme may be truncated at the N-terminal while still maintaining endo-beta-1,4-glucanase activity in full.

In the present context the expression "a cloned DNA sequence", either partial or complete, refers to a DNA sequence cloned by standard cloning procedure used in genetic engineering to relocate a segment of DNA from its natural location to a different site where it will be reproduced. The cloning process involves excision and isolation of the desired DNA segment, insertion of the piece of DNA into the vector molecule and incorporation of the recombinant vector into a cell where multiple copies or clones of the DNA segment will be replicated.

The "cloned DNA sequence" of the invention may alternatively be termed "DNA construct" or "isolated DNA sequence".

The DNA sequence may be of genomic, cDNA, or synthetic origin or any combinations of these. The endoglucanase or cellulase encoding part of the DNA sequence cloned into plasmid pSJ1678 present in *Escherichia coli* DSM 11203 and/or an analogue DNA sequence of the invention may be cloned from a strain of the archaeal species *Pyrococcus furiosus,* preferably the strain DSM 3638, producing the enzyme with cellulase, preferably endo-beta-1,4-glucanase, activity, or another or related organism as described further below.

Alternatively, the analogous sequence may be constructed on the basis of the DNA sequence of SEQ ID NO: 1 or the DNA sequence obtainable from the plasmid present in *Escherichia coli* DSM 11203, e.g be a sub-sequence thereof, and/or by introduction of nucleotide substitutions which do not give rise to another amino acid sequence of the cellulase encoded by the DNA sequence, but which corresponds to the codon usage of the host organism intended for production of the enzyme, or by introduction of nucleotide substitutions which may give rise to a different amino acid sequence (*i.e.* a variant of the cellulase of the invention).

It is believed that the DNA sequence of SEQ ID NO:1 corresponds to the DNA sequence obtainable from the plasmid present in *Escherichia coli* DSM 11203.

When carrying out nucleotide substitutions, amino acid changes are preferably of a minor nature, i.e. conservative amino acid substitutions which do not significantly affect the folding or the enzymatic activity of the protein, small deletions, typically of one to about 30 amino acids; small amino- or carboxyl-terminal extensions, such as an amino-terminal methionine residue, a small linker peptide of up to about 20-25 residues, or a small extension that facilitates purification, such as a poly-histidine tract, an antigenic epitope or a binding domain.

Examples of conservative substitutions are within the group of basic amino acids (such as arginine, lysine, histidine), acidic amino acids (such as glutamic acid and aspartic acid), polar amino acids (such as glutamine and asparagine), hydrophobic amino acids (such as leucine, isoleucine, valine), aromatic amino acids (such as phenylalanine, tryptophan, tyrosine) and small amino acids (such as glycine, alanine, serine, threonine, methionine). For a general description of nucleotide substitution, see e.g. Ford et al., (1991), Protein Expression and Purification 2, 95-107.

It will be apparent to persons skilled in the art that such substitutions can be made outside the regions critical to the function of the molecule and still result in an active polypeptide. Amino acids essential to the activity of the polypeptide encoded by the cloned DNA sequence of the invention, and therefore preferably hot subject to substitution, may be identified according to procedures known in the art, such as site-directed mutagenesis or alanine-scanning mutagenesis (cf. e.g. Cunningham and Wells, (1989), Science 244, 1081-1085). In the latter technique mutations are introduced at every residue in the molecule, and the resultant mutant molecules are tested for biological (*i.e.* cellulolytic) activity to identify amino acid residues that are critical to the activity of the molecule. Sites of substrate-enzyme interaction can also be determined by analysis of crystal structure as determined by such techniques as nuclear magnetic resonance analysis, crystallography or photoaffinity labelling (cf. e.g. de Vos et al., (1992), Science 255, 306-312; Smith et al., (1992), J. Mol. Biol. 224, 899-904; Wlodaver et al., (1992), FEBS Lett. 309, 59-64).

The endo-beta-1,4-glucanase encoded by the DNA sequence of the DNA construct of the invention may comprise a cellulose binding domain (CBD) existing as an integral part of the encoded enzyme, or a CBD from another origin may be introduced into the endo-beta-1,4-glucanase thus creating an enzyme hybride.In this context, the term "cellulose-binding domain" is intended to be understood as defined by Peter Tomme et al. "Cellulose-Binding Domains: Classification and Properties" in "Enzymatic Degradation of Insoluble Carbohydrates", John N. Saddler and Michael H. Penner (Eds.), ACS Symposium Series, No. 618, 1996. This definition classifies more than 120 cellulose-binding domains into 10 families (I-X), and demonstrates that CBDs are found in various enzymes such as cellulases, xylanases, mannanases, arabinofuranosidases, acetyl esterases and chitinases. CBDs have also been found in algae, e.g. the red alga *Porphyra purpurea* as a non-hydrolytic polysaccharide-binding protein, see Tomme et al., *op.cit.* However, most of the CBDs are from cellulases and xylanases, CBDs are found at the N and C termini of proteins or are internal. Enzyme hybrids are known in the art, see e.g. WO 90/00609 and WO 95/16782, and may be prepared by transforming into a host cell a DNA construct comprising at least a fragment of DNA encoding the cellulose-binding domain ligated, with or without a linker, to a DNA sequence encoding the endo-beta-1,4-glucanase and growing the host cell to express the fused gene. Enzyme hybrids may be described by the following formula:

$$CBD - MR - X$$

wherein CBD is the N-terminal or the C-terminal region of an amino acid sequence corresponding to at least the cellulose-binding domain; MR is the middle region (the linker), and may be a bond, or a short linking group preferably of from about 2 to about 100 carbon atoms, more preferably of from 2 to 40 carbon atoms; or is preferably from about 2 to to about 100 amino acids, more preferably of from 2 to 40 amino acids; and X is an N-terminal or C-terminal region of a polypeptide encoded by the DNA sequence of the invention.

The DNA sequence of the present invention can be cloned from the strain *Escherichia coli* DSM 11203 using standard methods e.g. as described by Sambrook et al., (1989), Molecular Cloning: A Laboratory Manual. Cold Spring Harbor Lab.; Cold Spring Harbor, NY.

The DNA sequence of the invention can also be cloned by any general method involving

- cloning, in suitable vectors, a DNA library from any organism, e.g. *Pyrococcus furiosus,* expected to produce the endo-beta-1,4-glucanase of interest,
- transforming suitable host cells with said vectors,
- culturing the host cells under suitable conditions to express any enzyme of interest encoded by a clone in the DNA library,
- screening for positive clones by determining any cellulolytic activity of the enzyme produced by such clones, and
- isolating the enzyme encoding DNA from such clones.

Alternatively, the DNA encoding a cellulase of the invention may, in accordance with well-known procedures, conveniently be cloned from a suitable source, such as any of the below mentioned organisms, by use of synthetic oligonucleotide probes prepared on the basis of the DNA sequence of SEQ ID NO:1 or the DNA sequence obtainable from the plasmid present in *Escherichia coli* DSM 11203.

## Homology of endo-beta-1,4-glucanase encoding DNA sequences

Homology search was performed using the DNA sequence of SEQ ID No. 1. The homology search showed that the most related DNA sequence was a gene encoding an endo-beta-1,4-glucanase from *Thermotoga neapolitana* (GenBank acc. no.U93354, Bok,J.D. and Eveleigh,D.E. Direct Submission to database Submitted (13-MAR-1997) Biochemistry and Microbiology, Cook College, Rutgers University, Lipman Drive, New Brunswick, NJ 08903, USA), to which gene the DNA sequence shown in SEQ ID NO 1 shows 34% identity.

The low homology identified using the homology search in the above demonstrates that the endo-beta-1,4-glucanase of the invention is distant from any known endo-beta-1,4-glucanase.

The DNA sequence homology referred to above is determined as the degree of identity between the two sequences indicating a derivation of the first sequence from the second. The homology may suitably be determined by means of computer programs known in the art such as GAP provided in the GCG program package (Needleman, S.B. and Wunsch, C.D., (1970), Journal of Molecular Biology, 48, 443-433 . Using GAP with the following settings for DNA sequence comparison: GAP creation penalty of 5.0 and GAP extension penalty of 0.3, provided in the GCG program package (Needleman, S.B. and Wunsch, C.D., (1970), Journal of Molecular Biology, 48, 443-453). Using GAP with the above settings for DNA sequence comparison, the DNA construct of the present invention comprises a DNA sequence exhibiting a degree of identity preferably of at least 40%, more preferably at least 45%, more preferably at least 50%, more preferable at least 60%, even more preferably at least 70%, especially at least 80%, in particular at least 95%, with the DNA sequence shown in SEQ ID No: 1 or the RNA sequence obtainable from the plasmid present in *Escherichia coli* DSM 11203.

**Hybridization**

Hybridization is intended to indicate that the analogous (partial) DNA sequence hybridizes to a nucleotide probe corresponding to the endo-beta-1,4-glucanase encoding part of the DNA sequence obtainable from the plasmid present in *Escherichia coli* DSM 11203 under certain specified conditions which are described in detail below.

Suitable conditions for determining hybridization between a nucleotide probe and a homologous DNA or RNA sequence involves presoaking of the filter containing the DNA fragments or RNA to hybridize in 5 x SSC (standard saline citrate) for 10 min, and prehybridization of the filter in a solutions of 5 x SSC Sambrook et al. 1989), 5 x Denhardt's solution (Sambrook et al. 1989), 0.5 % SDS and 100 μg/ml of denatured sonicated salmon sperm DNA (Sambrook et al. 1989), followed by hybridization in the same solution containing a random-primed (Feinberg, A. P. and Vogelstein, B. (1983) Anal. Biochem. 132:6-13), $^{32}$P-dCTP-labeled (specific activity > 1 x $10^3$ cpm/μg) probe for 12 hours at approx. 45°C. The filter is then washed two times for 30 minutes in 2 x SSC, 0.5 % SDS at a temperature of at least 55°C, more preferably at least 60°C, more preferably at least 65°C, even more preferably at least 70°C, especially at least 75°C.

Molecules to which the oligonucleotide probe hybridizes under these conditions are detected using a x-ray film.

**Homology to amino acid sequences**

Homology search was performed using the amino acid sequence shown in SEQ ID No: 2. The homology search showed that the most related protein was an endo-beta-1,4-glucanase from *Thermotoga neapolitana* (Acc. no. U93354, Bok,J.D. and Eveleigh,D.E. Direct Submission to database Submitted (13-MAR-1997) Biochemistry and Microbiology, Cook College, Rutgers University, Lipman Drive, New Brunswick, NJ 08903, USA), to which the amino acid sequence (SEQ ID No. 2) shows 39% identity.

Hydrophobic cluster analysis was performed using the protein sequence of the *Erwinia carotovora* cellulase (belonging to the Family 12 of glycosyl hydrolases) from SWISSPROT acession number P16630, together with the above mentioned *Thermotoga neapolitana* cellulase protein sequence and together with the protein sequence of SEQ ID NO:2 (the invention). This analysis showed that the protein having the protein sequence of SEQ ID NO:2 as well as the *Thermotoga neapolitana* cellulase belongs to the Family 12 of glycosyl hydrolases (Lemesle-Varloot L, Henrissat B, Gaboriaud C, Bissery V, Morgat A, and Mornon Jp Hydrophobic cluster analysis: Biochimie 72(8):555-574, 1990.)

The low homology identified using the homology search in the above demonstrates that the endo-beta-1,4-glucanase of the invention is distant from any known endo-beta-1,4-glucanase.

The amino acid sequence homology referred to above is determined as the degree of identity between the two sequences indicating a derivation of the first sequence from the second. The homology may suitably be determined by means of computer programs known in the art such as using FASTA of the GCG package using the following settings: Scoring matrix: GenRunData:blosum50.cmp, Variable pamfactor used Gap creation penalty: 12, Gap extension penalty: 2. provided in the GCG program package (Needleman, S.B. and Wunsch, C.D., (1970), Journal of Molecular Biology, 48, 443-453). Using FASTA with the above settings for amino acid sequence comparison, the amino acid sequence exhibits a degree of identity preferably of at least 450, more preferably at least 50%, more preferably at least 60%, more preferably at least 70%, more preferably at least 80%, especially at least 95%, with the amino acid sequence shown in SEQ ID No: 2 or the amino acid sequence of the polypeptide encoded by the endo-beta-1,4-glucanase encoding part of the DNA sequence of SEQ ID NO:1 or the DNA sequence obtainable from the plasmid present in *Escherichia coli* DSM 11203.

**Immunological cross-reactivity**

Antibodies to be used in determining immunological cross-reactivity may be prepared by use of a purified cellulolytic enzyme. More specifically, antiserum against the endo-beta-1,4-glucanase of the invention may be raised by immunizing rabbits (or other rodents) according to the procedure described by N. Axelsen et al. in: A Manual of Quantitative Immunoelectrophoresis, Blackwell Scientific Publications, 1973, Chapter 23, or A. Johnstone and R. Thorpe, Immunochemistry in Practice, Blackwell Scientific Publications, 1982 (more specifically p. 27-31). Purified immunoglobulins may be obtained from the antisera, for example by salt precipitation $((NH_4)_2 SO_4)$, followed by dialysis and ion exchange chromatography, e.g. on DEAE-Sephadex. Immunochemical characterization of proteins may be done either by Outcherlony double-diffusion analysis (O. Ouchterlony in: Handbook of Experimental Immunology (D.M. Weir, Ed.), Blackwell Scientific Publications, 1967, pp. 655-706), by crossed immunoelectrophoresis (N. Axelsen et al., supra, Chapters 3 and 4), or by rocket immunoelectrophoresis (N. Axelsen et al., Chapter 2).

## Microbial Sources

The taxonomy applied below are in accordance with the Maidak et al. (1996).

For the purpose of the present invention the term "obtained from" or "obtainable from" as used herein in connection with a specific source, means that the enzyme is produced or can be produced by the specific source, or by a cell in which a gene from the source have been inserted.

It is at present contemplated that the DNA encoding a cellulase of the invention may be obtained from an archaea, in particular a strain belonging to the phylum Euryarchaeota, preferably to the subdivision Thermococcoales, in particular a strain of the genus *Pyrococcus*, in particular a strain of *Pyrococcus furiosus.*

In a preferred embodiment, the cellulase of the invention is obtained from the strain *Pyrococcus furiosus* DSM 3638. It is at present contemplated that a DNA sequence encoding an enzyme homologous to the enzyme of the invention may be obtained from other archaeal strains, especially strains belonging to the genus *Pyrococcus.*

An isolate of a strain of *Pyrococcus furious* from which a cellulase of the invention can be derived is publicly available from Deutsche Sammlung von Mikroorganismen, DSM 3638.

Further, the plasmid pSJ1678 comprising the DNA sequence encoding the endo-beta-1,4-glucanase of the invention has been transformed into a strain of the *Escherichia coli* which was deposited by the inventors according to the Budapest Treaty on the International Recognition of the Deposit of Microorganisms for the Purposes of Patent Procedure at the Deutsche Sammlung von Mikroorganismen und Zellkulturen GmbH, Mascheroder Weg 1b, D-38124 Braunschweig, Federal Republic of Germany, on 11 October 1996 under the deposition number DSM 11203.

## Recombinant expression vectors

A recombinant vector comprising a DNA construct encoding the enzyme of the invention may be any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced intro a host cell, is integrated into the host cell genome in part or in its entirety and replicated together with the chromosome(s) into which it has been integrated.

The vector is preferably an expression vector in which the DNA sequence encoding the enzyme of the invention is operably linked to additional segments required for transcription of the DNA. In general, the expression vector is derived from plasmid or viral DNA, or may contain elements of both. The term, "operably linked" indicates that the segments are arranged so that they function in concert for their intended purposes, e.g. transcription initiates in a promoter and proceeds through the DNA sequence coding for the enzyme.

The promoter may be any DNA sequence which shows transcriptional activity in the host cell of choice and may be derived from genes encoding proteins either homologous or heterologous to the host cell.

Examples of suitable promoters for use in bacterial host cells include the promoter of the *Bacillus stearothermophilus* maltogenic amylase gene, the *Bacillus licheniformis* alpha-amylase gene, the *Bacillus amyloliquefaciens* alpha-amylase gene, the *Bacillus subtilis* alkaline protease gen, or the *Bacillus pumilus* xylosidase gene, or the phage Lambda $P_R$ or $P_L$ promoters or the E. coli lac, trp or tac promoters.

The DNA sequence encoding the enzyme of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like kanamycin, chloramphenicol, erythromycin, tetracycline, spectinomycine, or the like, or resistance to heavy metals or herbicides.

To direct an enzyme of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al., op.cit.).

**Host cells**

The DNA sequence encoding the present enzyme introduced into the host cell may be either homologous or heterologous to the host in question. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell which is capable of producing the present enzyme and includes bacteria, yeast, fungi and higher eukaryotic cells.

Examples of bacterial host cells which, on cultivation, are capable of producing the enzyme of the invention are gram-positive bacteria such as strains of *Bacillus,* such as strains of *B. subtilis, B. licheniformis, B. lentus, B. brevis, B. stearothermophilus, B. alkalophilus, B. amyloliquefaciens, B. coagulans, B. circulans, B. lautus, B. megatherium or B. thuringiensis,* or strains of *Streptomyces,* such as *S. lividans* or *S. murinus,* or gram-negative bacteria such as *Escherichia coli*. The transformation of the bacteria may be effected by protoplast transformation, electroporation, conjugation, or by using competent cells in a manner known per se (cf. Sambrook et al., supra).

When expressing the enzyme in bacteria such as *E. coli,* the enzyme may be retained in the cytoplasm, typically as insoluble granules (known as inclusion bodies), or may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the enzyme in gram-positive bacteria such as *Bacillus* or *Streptomyces* strains, the enzyme may be retained in the cytoplasm, or may be directed to the extracellular medium by a bacterial secretion sequence. In the latter case, the enzyme may be recovered from the medium as described below.

The yeast cell may be a strain of *Saccharomyces,* particularly *S. cerevisiae.* The fungal host cell may be a strain of the genus *Aspergillus, Fusarium* or *Trichoderma.*

**Method of producing a cellulolytic enzyme**

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

As defined herein, an isolated polypeptide (e.g. an enzyme) is a polypeptide which is essentially free of other polypeptides, e.g., at least about 20% pure, preferably at least about 40% pure, more preferably about 60% pure, even more preferably about 80% pure, most preferably about 90% pure, and even most preferably about 95% pure, as determined by SDS-PAGE.

The term "isolated polypeptide" may alternatively be termed "purified polypeptide".

When an expression vector comprising a DNA sequence encoding the enzyme is transformed into a heterologous host cell it is possible to enable heterologous recombinant production of the enzyme of the invention.

Thereby it is possible to make a highly purified or monocomponent cellulolytic composition, characterized in being free from homologous impurities.

In this context homologous impurities means any impurities (e.g. other polypeptides than the enzyme of the invention) which originate from the homologous cell where the enzyme of the invention is originally obtained from.

In the present invention the homologous host cell may be a strain of *Pyrococcus furiosus.*

The medium used to culture the transformed host cells may be any conventional medium suitable for growing the host cells in question. The expressed cellulolytic enzyme may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

## Enzyme compositions

In a still further aspect, the present invention relates to an enzyme composition comprising an enzyme exhibiting cellulolytic activity as described above.

The enzyme composition of the invention may, in addition to the cellulase of the invention, comprise one or more other enzyme types, for instance hemi-cellulase such as xylanase and mannanase, other cellulase or endo-beta-1,4-glucanase components, chitinase, lipase, esterase, pectinase, cutinase, phytase, oxidoreductase, protease, or amylase.

The enzyme composition may be prepared in accordance with methods known in the art and may be in the form of a liquid or a dry composition. For instance, the enzyme composition may be in the form of a granulate or a microgranulate. The enzyme to be included in the composition may be stabilized in accordance with methods known in the art.

Thermostable cellulases have potential uses in a lot of different industries and applications. Examples are given below of preferred uses of the enzyme composition of the invention. The dosage of the enzyme composition of the invention and other conditions under which the composition is used may be determined on the basis of methods known in the art.

The enzyme composition according to the invention may be useful for at least one of the following purposes.

## Uses

In the present context, the term "cellulosic material" is intended to mean fibers, sewn and unsewn fabrics, including knits, wovens, denims, yarns, and toweling, made from cotton, cotton blends or natural or manmade cellulosics (e.g. originating from xylan-containing cellulose fibers such as from wood pulp) or blends thereof. Examples of blends are blends of cotton or rayon/viscose with one or more companion material such as wool, synthetic fibers (e.g. polyamide fibers, acrylic fibers, polyester fibers, polyvinyl alcohol fibers, polyvinyl chloride fibers, polyvinylidene chloride fibers, polyurethane fibers, polyurea fibers, aramid fibers), and cellulose-containing fibers (e.g. rayon/viscose, ramie, hemp, flax/linen, jute, cellulose acetate fibers, lyocell).

In the textile industry cellulases are used for treatment of cotton and other cellulosic materials to obtain a surface treatment of the fibers which result in fabrics with altered properties such as reduced pilling tendency, fuzz removal, softer fabric, better hand or visual effects. Especially cellulases are used to produce a "stone-washed" look of denim. The use of cellulases at high temperatures makes it possible to create new looks of both denim and non denim cotton/cellulosic fabrics. The thermostable cellulases can be included in high temperature treatments of fabric which has not been possible before, cellulase wash over 80°C or under steam conditions with very low liquor ratio is possible which gives the potential of creating new looks.

The processing of cellulosic material for the textile industry, as for example cotton fiber, into a material ready for garment manufacture involves several steps: spinning of the fiber into a yarn; construction of woven or knit fabric from the yarn and subsequent preparation, dyeing and finishing operations. Woven goods are constructed by weaving a filling yarn between a series of warp yarns; the yarns could be two different types. Knitted goods are constructed by forming a network of interlocking loops from one continuous length of yarn. The cellulosic fibers can also be used for non-woven fabric.

The preparation process prepares the textile for the proper response in dyeing operations. The sub-steps involved in preparation are desizing (for woven goods), scouring and bleaching. A one step combined scour/bleach process is also used by the industry. Although preparation processes are most commonly employed in the fabric state; scouring, bleaching and dyeing operations can also be done at the fiber or yarn stage.

The processing regime can be either batch or continuous with the fabric being contacted by the liquid processing stream in open width or rope form. Continuous operations generally use a saturator whereby an approximate equal weight of chemical bath per weight of fabric is applied to the fabric, followed by a heated dwell chamber where the chemical reaction takes place. A washing section then prepares the fabric for the next processing step. Batch processing generally takes place in one processing bath whereby the fabric is contacted with approximately 8 -15 times its weight in chemical bath. After a reaction period, the chemicals are drained, fabric rinsed and the next chemical is applied. Discontinuous pad-batch processing involves a saturator whereby an approximate equal weight of chemical bath per weight of fabric is applied to the fabric, followed by a dwell period which in the case of cold pad-batch might be one or more days.

Woven goods are the prevalent form of textile fabric construction. The weaving process demands a "sizing" of the warp yarn to protect it from abrasion. Starch, polyvinyl alcohol (PVA), carboxymethyl cellulose, waxes and acrylic binders are examples of typical sizing chemicals used because of availability and cost. The size must be removed after the weaving process as the first step in preparing the woven goods. The sized fabric in either rope or open width form is brought in contact with the processing liquid containing the desizing agents. The desizing agent employed depends upon the type of size to be removed. For PVA sizes, hot water or oxidative processes are often used. The most common sizing agent for cotton fabric is based upon starch. Therefore most often, woven cotton fabrics are desized by a combination of hot water, the enzyme α-amylase to hydrolyze the starch and a wetting agent or surfactant. The cellulosic material is allowed to stand with the desizing chemicals for a "holding period" sufficiently long to accomplish the desizing. The holding period is dependent upon the type of processing regime and the temperature and can vary from 15 minutes to 2 hours, or in some cases, several days. Typically, the desizing chemicals are applied in a saturator bath which generally ranges from about 15°C to about 55°C. The fabric is then held in equipment such as a "J-box" which provides sufficient heat, usually between about 55°C and about 100°C, to enhance the activity of the desizing agents. The chemicals, including the removed sizing agents, are washed away from the fabric after the termination of the holding period.

In order to ensure a high whiteness or a good wettability and resulting dyeability, the size chemicals and other applied chemicals must be thoroughly removed. It is generally believed that an efficient desizing is of crucial importance to the following preparation processes: scouring and bleaching.

The scouring process removes much of the non-cellulosic compounds naturally found in cotton. In addition to the natural non-cellulosic impurities, scouring can remove dirt, soils and residual manufacturing introduced materials such as spinning, coning or slashing lubricants. The scouring process employs sodium hydroxide or related causticizing agents such as sodium carbonate, potassium hydroxide or mixtures thereof. Generally an alkali stable surfactant is added to the process to enhance solubilization of hydrophobic compounds and/or prevent their redeposition back on the fabric. The treatment is generally at a high temperature, 80°C - 100°C, employing strongly alkaline solutions, pH 13-14, of the scouring agent. Due to the non-specific nature of chemical processes not only are the impurities but the cellulose itself is attacked, leading to damages in strength or other desirable fabric properties. The softness of the cellulosic fabric is a function of residual natural cotton waxes. The non-specific nature of the high temperature strongly alkaline scouring process cannot discriminate between the desirable natural cotton lubricants and the manufacturing introduced lubricants. Furthermore, the conventional scouring process can cause environmental problems due to the highly alkaline effluent from these processes. The scouring stage prepares the fabric for the optimal response in bleaching. An inadequately scoured fabric will need a higher level of bleach chemical in the subsequent bleaching stages.

The bleaching step decolorizes the natural cotton pigments and removes any residual natural woody cotton trash components not completely removed during ginning, carding or scouring. The main process in use today is an alkaline hydrogen peroxide bleach. In many cases, especially when a very high whiteness is not needed, bleaching can be combined with scouring.

It is contemplated that the scouring step can be carried out using the endo-beta-1,4-glucanase or endo-beta-1,4-glucanase preparation of the present invention in combination with a few other enzyme activities at a temperature of about 90°C - 100°C and a pH of about 7-9, thus substituting or supplementing the highly causticizing agents.

Use of thermostable cellulases in industrially cleaning processes makes it possible to make an easier cleaning process when the unwanted material to be removed is based on cellulosic matter. Examples are cleaning of ultra filtration membranes, pipes and the like in food/feed industry.

Incorporation of thermostable cellulase in heat extruded plast and polymer materials with cellulose filler, will result in higher degradeability of these materials in nature.

Lignocellulosic materials make up a big part of agricultural and forestry waste and in paper which is very dominant in municipal waste. This waste is typically burned, which from an energy point of view is an enormous waste of resources. A lot of work has been assigned to the task of developing an effective and economic process for conversion of this biomass to sugars that can be fermented to produce e.g. alcohol for use as fuel. Proposed processes for conversion of lignocellulosics to sugars include the use of cellulases but very high dosages are needed which make them unrealistic in big industrial scale from an economical point of view. The use of thermostable cellulases with liquefying properties makes such a bioconversion process more realistic. Processing at high temperature opens the cell wall structure in a lot of plant materials and makes the cellulose more accessible for enzyme attack.

In the industrial production of alcohol from different kind of grains, the traditional process includes liqu faction with alpha amylase at temperatures around 80-100°C. Inclusion of cellulase in this step will increase the yield of fermentable sugars. This preliquefaction of cellulose will also make the inclusion of traditional cellulases in the following process realistic, due to a higher hydrolysis rate than without preliquefaction.

Predigestion of grains; rye, barley, maize etc for feed production is another potential use of thermostable cellulase, this in order to increase digestibility of the feed in the animal.

Coffee extraction for production of instant coffee is carried out at temperatures 85-150°C in a battery of percolation columns. Water pass coutercurrent from the most extracted cell to the one just filled with fresh coffee. The operation temperature for the cells with the fresh coffee is approx. 100°C. Inclusion of thermophile cellulases at this point will increase capacity of the columns since the soluble matter is released easier when the cell wall structure is opened.

Inclusion of cellulases in other traditional high temperature processes for extraction of oil or aroma/flavour compounds from natural plant sources will in the same way as for coffee extraction increase capacity or yield. An example is extraction of palm oil or palm kernel oil which is an aqueous high temperature process.

The cellulases of the invention are also useful for degradation, i.e. viscosity-reduction, of aqueous solutions of hydrocolloid cellulose derivatives (CMC, HEC, HPC and other conventionally used cellulose derivatives) used within the oil industry in drilling and in enhanced oil recovery (EOR) by polymer flooding.

## MATERIALS AND METHODS

### Deposited organisms:

*Pyrococcus furiosus* DSM 3638, comprises the cellulase encoding DNA sequence of the invention.

*Escherichia coli* DSM 11203 containing the plasmid comprising the DNA sequence encoding the cellulolytic enzyme of the invention, in the cloning vector pSJ1678.

### Other strains:

*E. coli* strain: *E. coli* SJ2 (Diderichsen et al., 1990) Electrocompetent cells prepared and transformed using a Bio-Rad GenePulser™ as recommended by the manufacturer.

*B. subtilis* A164. This strain is a derivative of the B.subtilis ATCC 6051a, being sporulation deficient and having had the *apr* and *npr* genes disrupted. The disruptions were performed essentially as described in Sonenshein et al. (1993). Competent cells were prepared and transformed as described by Yasbin et al. (1975).

### Plasmids:

pBluescript II KS- (Stratagene, U.S.A.), and pSJ1678 (see WO 94/19454).

pUB110: Plasmid described in McKenzie et al. (1986) and McKenzie et al. (1987).

pMUTIN-4-MCS: Plasmid can be obtained from Laboratoire de Genetique Microbienne, Institut National de la Recherche Agronomique, 78352 Jouy en Josas - CEDEX, France.

### General molecular biology methods:

DNA manipulations and transformations were performed using standard methods of molecular biology (Sambrook et al. (1989); Ausubel, F. M. et al., 1995; Harwood, C. R. Et al., 1990).

Enzymes for DNA manipulations were used according to the specifications of the suppliers.

### Isolation of the DNA sequence encoding the cellulytic enzyme of the invention:

The DNA sequence encoding the endo-beta-1,4-glucanase of the invention, can be obtained from the deposited organism *E. coli,* DSM 11203, by extraction of plasmid DNA by methods known in the art (Sambrook et al. (1989)).

### Cloning of the endo-beta-1,4-glucanase gene

### Genomic DNA preparation:

Strain *Pyrococcus furiosus,* DSM 3638, was propagated in a fermentor for 14-24 hours at 90 °C on the medium as described below.

Composition of the medium for strain DSM 3638:

1.3 g $(NH_4)_2SO_4$, 0.25 g $MgSO_4$ $7H_2O$, 30 g NaCl, 1.4 g $KH_2PO_4$, 50 mg $CaCl_2$, 38 mg $FeSO_4$ $7H_2O$, 5 μmol $Na_2SeO_3$ $5H_2O$ , 10 ml Trace elements[#], 1 g Tryptone, 1 g Yeast extract, 1 g Starch, 1 mg Resazurin, 0.5 g Cysteine HCl, $H_2O$ to 1 1.

#Trace element solution:

0.1 g $MnCl_2$ $4H_2O$, 0.2 g $COCl_2$ $6H_2O$, 0.1 g $NiCl_2$ $6H_2O$, 0.1 g $ZnCl_2$, 50 mg $CaCl_2$ $2H_2O$, 50 mg $CuSO_4$ $2H_2O$, 50 mg $Na_2MoO_4$ $2H_2O$, $H_2O$ to 1 l. Adjust to pH 6.2-6.5, gassing with $N_2/CO_2$ (4:1).

Cells were harvested, and genomic DNA isolated by the method described by Pitcher et al., 1989.

## Genomic library construction:

Genomic DNA was partially digested with restriction enzyme Sau3A, and size-fractionated by electrophoresis on a 0.7 % agarose gel. Fragments between 2 and 7 kb in size were isolated by electrophoresis onto DEAE-cellulose paper (Dretzen, G., et al., 1981).

Isolated DNA fragments were ligated to *Bam*HI digested pSJ1678 plasmid DNA, and the ligation mixture was used to transform *E. coli* SJ2.

Cells were plated on LB agar plates containing 0.1% CMC (Sodium-Carboxy-Methyl-Cellulose, Aqualon, France) and 9 μg/ml Chloramphenicol to give 500-1000 c.f.u./plate and incubated overnight at 37°C.

## Identification of positive clones by activity:

After inkubation the colonies were replica plated onto a set of LB+CAM agar plates and then further incubated at 37°C for approx. 20 hours. An overlayer containing 0.1% CMC, 1% HSB agarose in an appropriate buffer pH 7 was poured onto the replica plates and incubated for approx. 20 hours at 65°C. Endo-beta-1,4-glucanase positive colonies were identified by staining with a 0.1% aqueous solution of Congo Red (SIGMA, USA) followed by washing in 2 M NaCl. Yellowish halos appeared at positions where endo-beta-1,4-glucanase positive clones were present

Cells from enzyme-positive colonies were spread for single colony isolation on agar, and an enzyme-producing single colony was selected for each of the endo-beta-1,4-glucanase-producing colonies identified.

## Characterization of positive clones:

From the restreaking plates the endo-beta-1,4-glucanase positive clones were obtained as single colonies, and plasmids were extracted. Phenotypes were confirmed by retransformation of *E. coli* SJ2, and plasmids characterized by restriction digests.

## Sequencing of the Endo-beta-1,4-glucanase encoding DNA:

The endo-beta-1,4-glucanase gene was characterized by DNA sequencing by primerwalking, using the Taq deoxyterminal cycle sequencing kit (PerkinElmer, USA), fluorescent labeled terminators and appropriate oligonucleotides as primers. Analysis of the sequence data was performed according to Devereux et al.(1984) Nucleic Acids Res. 12, 387-395. The sequence corresponds to the DNA sequence shown in SEQ ID No: 1.

## Media

TY and LB agar (as described in Ausubel et al., 1995)

**Hybridization conditions** (to be used in evaluating property ii) of the DNA construct of the invention):

Suitable conditions for determining hybridization between a nucleotide probe and a homologous DNA or RNA sequence involves presoaking of the filter containing the DNA fragments or RNA to hybridize in 5 x SSC (standard saline citrate) for 10 min, and prehybridization of the filter in a solution of 5 x SSC (Sambrook et al. 1989), 5 x Denhardt's solution (Sambrook et al. 1989), 0.5 % SDS and 100 μg/ml of denatured sonicated salmon sperm DNA (Sambrook et al. 1989), followed by hybridization in the same solution containing a random-primed (Feinberg et al. (1983)), $^{32}$P-dCTP-labeled (specific activity > 1 x $10^9$ cpm/μg) probe for 12 hours at approx. 45°C. The filter is then washed two times for 30 minutes in 2 x SSC, 0.5 % SDS at a temperature preferably of at least 55°C, more preferably at least 60°C, more preferably at least 65°C, even more preferably at least 70°C, especially at least 75°C.

The nucleotide probe to be used in the hybridization is the endo-beta-1,4-glucanase encoding part of the DNA sequence obtainable from the plasmid present in *Escherichia coli* DSM 11203.

**Immunological cross-reactivity:**

Antibodies to be used in determining immunological cross-reactivity may be prepared by use of a purified endo-beta-1,4-glucanase. More specifically, antiserum against the endo-beta-1,4-glucanase of the invention may be raised by immunizing rabbits (or other rodents) according to the procedure described by N. Axelsen et al., 1973, or A. Johnstone et al., 1982. Purified immunoglobulins may be obtained from the antisera, for example by salt precipitation (($NH_4)_2$ $SO_4$), followed by dialysis and ion exchange chromatography, e.g. on DEAE-Sephadex. Immunochemical characterization of proteins may be done either by Outcherlony double-diffusion analysis (Ouchterlony, 1967), by crossed immunoelectrophoresis (N. Axelsen et al., supra, Chapters 3 and 4), or by rocket immunoelectrophoresis (N. Axelsen et al., Chapter 2).

**Construction of expression vector:**

In the following is described the construction of an expression vector. The expression vector used in example 1 for expressing the DNA sequence encoding the endo-beta-1,4-glucanase of the invention is constructed in a similar manner.

The following description (written in Italic) has been included in order to describe the construction of the expression vector used further below for expressing the DNA sequence encoding the cellulolytic enzyme of the invention, i.e. SEQ ID NO:1:

The pMUTIN4MCS is an E. coli plasmid having a hybrid promoter SPAC (Yansura et al.(1984)) and a lacZ gene under transcriptional control of this promoter. Furthermore the plasmid contains the lacl gene under control of a Bacillus spe. penP Promoter. Thus when in B. subtilis the lacl will be expressed and bind to the operator of the SPAC promoter-operator and this will inhibit transcription of the downstream sequence. The Promoter is the same as in Yansura et al. however the operator was modified into a perfect palindrome. A HindIII site just downstream of the Promoter-operator region made it possible to disrupt the lacZ gene by insertion of another gene, namely the endo-beta-1,4-glucanase gene of this invention. Thus leaving the expression of the endoglucanse under the control of the SPAC promoter-Operator.

In the E. coli plasmid pMUTIN4MCS a ribosome binding site (RBS) and a signal peptide with a SacII site for cloning purposes, were cloned as a HindIII-Sacl fragment. Thus establishing the following ribosome binding site and signal peptide encoding DNA sequence:

```
HindIII

                RBS

5'-AAGCTTGTTACACATTGAAAGGGGAGGAGAATCATGAAACAACAAAAACGGCTTT-

ACGCCCGATTGCTGACGCTGTTATTTGCGCTCATCTTCTTGCTGCCTCATTCTGCAGCCG-

        NotI      SacI

CGGCA GCGGCCGC GAGCTC-3'
```

Written in italic is the DNA sequence encoding a Bacillus spe. signal peptide which when fused to another DNA sequence encoding the mature part of a protein will direct this to the exterior of a Bacillus spe. cell.

The sequence above encodes the HindIII site of pMUTIN4MCS directly followed by a Bacillus subtilis RBS and a DNA sequence encoding a Bacillus spe. signal peptide ending with an artificially introduced SacII site which is followed by a NotI and a Sacl restriction site. The plasmid was established in E. coli SJ2 by electroporation and plating on LB-agar plates with 100 μg/ml of ampicilin and incubating the cells overnight at 37°C. The resulting plasmid was termed pMUTIN4-Signal SacII-NotI.

The thermostable endo-beta-1,4-glucanase gene was cloned as a SacII-EagI digested PCR fragment.

The PCR fragment was obtained using the HiFi Expand PCR kit from Boehringer Mannheim and reaction was performed as recommended by the manufacturer. The DNA fragment was amplified from the plasmid pDSM11201. The thermostable endo-beta-1,4-glucanase gene was originally cloned from Dictyoglomus spe. DSM6262 and deposited as an E. coli clone (DSM 11201) harbouring a plasmid with the endo-beta-1,4-glucanase gene. The two PCR primers used were as follows:

#30519:
5'-CATTCTGCAGCCGCGGCACAAACTCCAAAATACAAAGACGC-3'

#30637:
5'-CATGACACGGCCGATTATTTAAGCTCAATATCAAAATTTGAG-3'

The PCR fragment and the pMUTIN4-Signal SacII-NotI were digested with SacII-EagI, ligated together and used to transform E. coli SJ2 by electroporation and plating on LB-agar plates with 100 μg/ml of ampicillin and incubating the cells overnight at 37°C.. The resulting plasmid in SJ2 was termed pMB447A.

After having cloned the endo-beta-1,4-glucanase gene in fusion with the signal peptide from above (in the SacII-Not/EagI site) the following open reading frame resulted under the transcriptional control of the SPAC promoter-operator:

ATGAAACAACAAAAACGGCTTTACGCCCGATTGCTGACGCTGTTATTTGCGCTCATCTTCTTGC
TGCCTCATTCTGCAGC
CGCGGCACAAACTCCAAAATACAAAGACGCATTTATACTTAAAGCACCTTCCTCAGGCGATGTC
ACAACTAAAAATCTTC
CTCTCACCTTAGAACTCAACTTTTGGAATATTGCAAACTATGAAGGAAATACATGGATGGCATT
TTATAAAGAAGAAGAT
ACTGTTGAATATTATGCCGACATAAAAAACATAGTACTTAAGGATAAAAATTCATGGGTACATG  .
GATATCCTGAAGTCTA
CTATGGGTACAAACCATGGGCTGGCCATGGGAATTCCATTGAGAAATTAGCTCTTCCTAAAAAG
GTATCAGAATTTCCAG
ACGTTCTCTTCAATCTAAAATACAACATATGGTACGAGAAGAATCTTCCTATAAATTTTGCTAT
GGAAACATGGATAACA
AAAGAACCCTATCAGAAACCGTTACTTCAGGGGATATAGAGATGATGGTATGGCTATATGCTA
ATAGACTTTCTCCTGC
AGGGCGAAAGGTAGGAGAAGTAAAAATACCTATCATCCTAAACGGTAATCAAAAAGACATTATC
TGGGAAGTATATCTTT
CCCCTATGAGCTGGGACTACGTGGCCTATAAATCAAAAGAAAATATTCTTCAAGGACAGGTAAA
AATACCAATAAATGAA
TTTTTGAAACACCTAAGAACAATTTTAGCCAACAATCCAAGTAGAATAACCCCAGAGAAATTTG
ATCAGATGTATGTGAC
AGTCTGGGAAATTGGAACAGAATTTGGCGATCCATATACTACTGAGGCAAAATTTGGATGGACT
TTCTCAAATTTTGATA
TTGAGCTTAAATAA

and the derived protein:

```
MKQQKRLYARLLTLLFALIFLLPHSAAAAQTPKYKDAFILKAPSSGDVTTKNLPLTLELNFWNI
ANYEGNTWMAFYKEED
TVEYYADIKNIVLKDKNSWVHGYPEVYYGYKPWAGHGNSIEKLALPKKVSEFPDVLFNLKYNIW
YEKNLPINFAMETWIT
KEPYQKTVTSGDIEMMVWLYANRLSPAGRKVGEVKIPIILNGNQKDIIWEVYLSPMSWDYVAYK
SKENILQGQVKIPINE
FLKHLRTILANNPSRITPEKFDQMYVTVWEIGTEFGDPYTTEAKFGWTFSNFDIELK.
```

In order to be able to propagate the pMB447A in Bacillus subtilis the E. coli plasmid was fused to a derivative of pUB110 (a plasmid propagateable in B.subtilis): In the NciI site of pUB110 a SacI and NotI site were introduced using a polylinker the resulting insert had the following sequence:
*CCCGGGAGCTCGCGGCCGCCCCGG*

The two NciI sites are underlined in-between these are the SacI and EagI (NotI) sites.

This plasmid was then SacI and EagI digested and ligated to SacI EagI digested pMB447A, the ligation was used to transform Bacillus subtilis A164. Clones were established and grown overnight on LBPG-10 Kana AZCL-HE-cellulose plates. Next day these plates were incubated at 70°C for 5 hours and the appearance of blue haloes indicated positive expression of the thermostable endo-beta-1,4-glucanase.

When analysing the plasmid DNA isolated from the clone MB505 (DSM 11903) it was apparent that the plasmid had underwent recombination and the resulting plasmid was smaller than the expected plasmid size of 12.5 kb. Thus it appeared that the plasmid had lost most of the E. coli plasmid pMUTIN 4 Signal SacII-NotI, resulting in a plasmid of the approximate size of 5.5 kb.

The resulting plasmid had the following essential features: The endo-beta-1,4-glucanase encoded on the plasmid of DSM 11903 was positively expressed without having to add IPTG and the plasmid conferred resistance to 10 $\mu$g/ml of Kanamycin.

**CMCU assay at 90°C (determination of endo-beta-1,4-glucanase activity):**

One CMCU unit is defined as the amount of enzyme which release the equivalent to 1 mmol of glucose per min under the following standard conditions (CMC assay at 90 °C): The enzyme is incubated for 20 min. in a 0.75% CMC (7L from Hercules) solution in a 0.1 M sodium barbiturate buffer pH 8.5. After incubation the increase in reducing end groups is determined using PHBAH (SIGMA H-988253H7704 (p-HYDROXY BENZOIC ACID HYDRAZIDE)); and with a glucose standard the formation of glucose equivalent end groups per min is calculated. (Lever, M. (1972) A new reaction for colometric determination of carbohydrates. Anal. Biochem. vol 47, page 273-279).

**p-Nitrophenyl-beta-D-cellobioside assay:**

One PNPU unit is defined as the amount of enzyme which release one mmole of p-Nitrophenol per minute from p-Nitrophenyl-beta-D-cellobioside (Sigma) under standard assay conditions (cf. below).

Method:

Steady state kinetic, direct detection of the product p-Nitrophenol, it gives a yellow color, which is detected at 405 nm. The activity is measured as the increase of absorbency, the linear part of the curve is used to determine the slope (AU/sec). The activity is calculated using an absorbance of p-Nitrophenol in phoshate buffer pH 7.5: Absorbance in 1 cm cuvette, 1mM of 0.018 at 405 nm (0.014 at 420 nm).

Assay conditions:

475 ml 10 mM p-NP-beta-D-cellobioside in 0.1 M Na-phosphate buffer, pH 7.5; 25 ml Enzyme solution

Procedure:

The measurement is made on HP 8452A Diode Array Spectrophotometer thermostatically controlled to 70 °C in a 0.75 ml cuvette, 1 cm width.

Absorbance at 405 nm is measured in 300 sec with a measurement every 20 sec.

The following non-limiting examples illustrate the invention.

**EXAMPLE 1**

**A. Cloning of an endo-beta-1,4-glucanase from *Pyrococcus furiosus* DSM 3638**

A library from *Pyrococcus furiosus* DSM 3638 was constructed in *E. coli* and screened as described in Materials and Methods. One positive transformant isolated was DSM 11203 containing the plasmid pSJ1678 comprising the DNA sequence encoding the cellulolytic enzyme of the invention, i.e. SEQ ID NO:1.

**B. Cloning of the *Pyroccocus furiosus* endo-beta-1,4-glucanase gene from the deposited *E. coli* clone DSM 11203.**

From the DNA SEQ ID NO:1 a set of PCR primers were designed. These primers were designed in order to obtain a fragment encoding the DNA sequence corresponding to the most likely mature form of the endo-beta-1,4-glucanase of the invention. The fragment was cloned as a SacII-EagI fragment in the vector pMB505 described above.

The PCR was performed using the Expand High Fidelity PCR system (#1732650, Boehringer Manheim, GmbH). Reactions were set up using the buffer 2 system together with 200 $\mu$M of each the four dNTP's (dATP, dCTP, dGTP and dTTP), 0.75 $\mu$l Enzyme Mix and 250 nM of each of the following PCR primers:

```
#105847
5'- CAT CAT TCT GCA GCC GCG GCA ATA TAT TTT GTA GAA AAG TAT CAT
ACC-3'
```

```
#105871
5'- GCA CAA GCT TGC GGC CGC TTA GGA AAT AAG AGG TCT ATC TAG-3'
```

The PCR reaction was performed in 50 $\mu$l volumes and using 100-200 ng of plasmid obtained from the *E. coli* clone DSM 11203. PCR reactions were temperature cycled in an Landgraf thermocycler (Hans Landgraf, GmbH). The following temperature cycling profile was used:

* 1 cycle at 94°C for 60 sec.
* 10 cycles of (94°C for 15 sec, 60°C for 60 sec, 72°C for 60 sec)
* 20 cycles of (94°C for 15 sec, 60°C for 30 sec, 72°C for 60 sec add 20 sec per cycle to the elongation step).
* 1 cycle at 72°C for 300 sec.

The time used to go from one temperature to another was set to 1 sec (for this thermocycler that means as fast as possible (aproximately 10 sec).

After ending the cycling the 50 $\mu$l reactions were purified using the Qiagen PCR purification kit according to the manufacturers instructions (Qiagen, GmbH).

After purification, the PCR fragment and plasmid pMB505 (obtained from *Bacillus subtilis* clone DSM 11903 using the plasmid prep kit from Qiagen #27106) were both cut with the restriction enzymes SacII and EagI. The digested DNA was run on a 0.7% Agarose gel stained with EtBr and the DNA fragments of interest were cut from the gel and purified using the Qiagen kit #28706 according to the manufacturers instructions.

After elution of the DNA from the purification coloumns some of the PCR fragment (aproximately 100 ng) and some of the vector (aproximately 400 ng) were ligated in 20 ul volumen using 1 unit of the T4-DNA ligase in its appropriate buffer from Boehringer Mannheim (GmbH), and incubating the ligation mixture at 16°C overnight. Half of this ligation was used to transform *Bacillus subtilis* A164. After transformation cells were plated on LBPG-AZCL-HE-cellulose plates with 10 $\mu$g/ml of Kanamycin and incubated overnight at 37°C. Single colonies were choosen, restreaked on fresh plates for incubation overnight at 37°C. Then single colonies of several clones were grown in 10 ml TY at 37°C overnight and plasmids were isolated and analyzed by restriction enzyme digestion using different specific enzymes.

40 $\mu$l of culture broth from the overnight liquid cultures were transferred to holes punctured in LBPG-AZCL-HE-cellulose plates. This plate were then incubated at 80°C for 6 hours and clear blue halos around holes indicated that the *Pyrococcus* furiosus endo-beta-1,4-glucanase of DSM 3638 and the subcloned gene from DSM 11203 was positively expressed in *Bacillus subtilis*.

EP 0 972 016 B1

One such positively expressed clone was termed MB544. MB544 was cultivated for 5 days in 500 ml shake flasks with two baffles and 100 ml of BPX media at 37°C at 300 rpm. This culture broth was used to purify the expressed endo-beta-1,4-glucanase.

Media

**LB agar** (as described in Ausubel, F. M. et al. (eds.) "Current protocols in Molecular Biology". John Wiley and Sons, 1995). **LBPG** is LB agar supplemented with 0.5% Glucose and 0.05 M potassium phosphate, pH 7.0 **AZCL-HE-cellulose** is added to LBPG-agar to 1%. AZCL-HE-cellulose is from Megazyme, Australia. **BPX media** is described in US 5,695,976 and the corresponding EP-B-0 506 780.

**EXAMPLE** 2

**Purification and characterization of the expressed *Pyroccocus furiosus* endo-beta-1,4-glucanase clone**

A. Purification and characterization

3600 ml shake flask culture fluid from *Bacillus* with the clone descibed in example (1) was received and 3200 ml of the culture broth was adjusted to pH 5.5 with HCl followed by treatment with 150 ml of a cationic flocculation agent under stirring at room temperature and subsequently 300 ml of a 0.1% solution of an anionic flocculation agent. The flocculated material was separated by centrifugation using a Sorval RC 3B 10.000 rpm for 30 min. The supernatant was clarified using a Whatman glass filter number F. A total of 3400 ml was obtained with an activity of 8 CMCU per ml (activity determination at 90°C and pH 8.5).
The liquid was applied to 3000 ml DEAE A-50 Sephadex equilibrated in 50 mM Acetate buffer pH 5.5. The non-bound material contained total 18000 CMCU.
The non bound material was concentrated to 1.4 litres on a filtron with a cut off at 10 kDa. Two times 400 ml liquid adjusted with ammonium acetate to 1 M was then applied to a 400 ml Butyl Toyopearl column equilibrated with 1 M ammonium acetate buffer pH 6.5. The bound enzyme was eluted using a deionized water. Total 17000 CMCU units was obtained.
4000 CMCU units was formulated with glycerol to a final concentration of 40% and used for applications trials.

B. Characterization

The endo-beta-1,4-glucanase of the invention has a molar extinction coefficient of 84350 at 280 nm, an apparant molecular weight (MW) of 34 kDa, and pI 4.8.
The pure endo-beta-1,4-glucanase has activity on p-Nitrophenyl-beta-D-cellobioside (Sigma), CMC, HE cellulose (from Megazyme) and acid swollen cellulose.

Temperature activity profile of the endo-beta-1,4-glucanase

The endo-beta-1,4-glucanase was incubated in the CMCU assay at pH 7.5 in 0.1 M Mops buffer at different temperatures and the activity after 20 min. incubation was determined as described above. Incubations above 100°C were not performed. The amount of reducing sugar obtained at this temperature was the highest and used for calculation of the relative activity at lower temperatures.

| Temp. | Rel. activity |
| --- | --- |
| 100° C | 96% |
| 90° C | 100% |
| 80° C | 65% |
| 70° C | 32% |
| 60° C | 13% |

pH activity profile

The endo-beta-1,4-glucanase was incubated in the CMCU assay at 90°C using different buffers in the pH interval of 4.5 to 11 and the activity after 20 min. incubation was determined as described above.

17

Buffers; pH 4.5, 5.0 and 5.5; 0.1 M Na-acetate
pH 6.0; 0.1 M Na-MES
pH 6.5, 7.0 and 7.5; 0.1 M Na-MOPS
pH 8.0 and 8.5; 0.1 M EPPS
pH 9.0, 9.5, 10.0, 10.5 and 11.0; 0.1 M Na-glycine

More than 50% relative activity was obtained in the interval of pH 5.0 to 9.5.

## EXAMPLE 3

**Biopolishing of Cotton Knit with the inventive Endo-beta-1,4-glucanase from *Pyrococcus* in Pad-Steamer System**

### Experimental

*Fabric and Solution Preparation:* Test Fabric #460, bleached knitted cotton interlock (from Test Fabrics, Inc., Middlesex, NJ), was cut to about 20x30 cm swatches and conditioned in climate room (65$\pm$2 % relative humidity; 70$\pm$3°F /21$\pm$2°C temperature) for at least 24 hours. The swatches all weighed between 11.8 to 12.6g. Solutions were made to have 0, 2 CMCU/ml with buffers and enzyme. Buffers are made by dissolving 1.5g Tris(Hydroxymethyl) aminomethane in 500ml deionized water, adjust pH to 8.9 and 7.3 with HCl. The endo-beta-1,4-glucanase produced as described in example 1 and 2 (Batch MB473, #9730) was used.

*Padding:* swatches were padded with enzyme solutions with the Mathis Pad-steamer range (Werner Mathis USA Inc. Concord, NC). The wet pickup were calculated from fabric weights before and after padding, and 100$\pm$5 % wet pickup was obtained. Two swatches were padded for each pH solutions. At each pH, cellulase concentrations are 0 and 2 CMCU/ml.

*Incubation:* Mathis pad steamer range was preheated to temperature of 90°C and relative humidity (RH) of 100%. After padded with solution, a swatch was hung in the steamer chamber manually. Opening the chamber door of the pad steamer range decreases temperature and RH. However, the original temperature and RH can be reached within 2 minutes. All swatches were treated at the same time for 90 minutes.

*Rinsing:* After swatches were taken out from steamer chamber, they were rinsed in de-ionized water for 5 minutes. Then they were air dried and equilibrated in climate room for at least 24 hours before evaluation.

*Evaluation:* Pilling resistance measured by pilling note according to ASTM D 4970 - 89. Nu-Martindale Abrasion and Pilling Tester (James H. Heal & Co. Ltd., UK) is used. Pilling note after 125 revolutions was recorded. A range of pilling note 1-5 was given by comparing with standard photographs where 1 is heavily pilled and 5 has no pill.

Burst strength loss was measured according to ASTM D3786 - 87 method. Mullen Burst Tester (Model C) (from B.F. Perkins, Chicopee, MA) is used.

### Results and Conclusions

Pilling note was measured twice and burst strength was measured at least seven times. The data are shown in Table 1. No statistically significant strength loss was detected for all endo-beta-1,4-glucanase treated swatches when compared with no enzyme control at the same pH. At pH 7.3, the swatches treated with 0 and 2 CMCU/g fabric show pilling note of 3 and 4 after 125 revolutions, respectively. At pH 8.9, the swatches treated with 0 and 2 CMCU/g fabric show pilling note of 2.5 and 4 after 125 revolutions, respectively. These results show a significant difference. Compared to 0 CMCU/g fabric treated fabric, endo-beta-1,4-glucanase treated fabric has better hand and appearance.

It can be concluded that the family 12 endo-beta-1,4-glucanases of the present invention are able to show good biopolishing effects with little fabric strength loss in Pad Steamer range from pH 7.3 to 8.9, 90°C. The amount of endo-beta-1,4-glucanase for giving improved pilling note can be as little as 2 CMCU/g fabric. A better appearance and fabric hand are also achieved.

Table 1

| | Endo-glucanase | Burst Strength (psi) | | Pilling Note (125 rev.) | |
|---|---|---|---|---|---|
| | (CMC/g fabric) | Average | STDEV | Average | STDEV |
| pH 7.3 | 0 | 110 | 4.9 | 3 | 0 |
| | 2 | 109 | 4.3 | 4 | 0 |
| pH 8.9 | 0 | 113 | 3.9 | 2.5 | 0 |

(continued)

|  | Endo-glucanase | Burst Strength (psi) | | Pilling Note (125 rev.) | |
|---|---|---|---|---|---|
|  | (CMC/g fabric) | Average | STDEV | Average | STDEV |
|  | 2 | 114 | 4.8 | 4 | 0 |
| STDEV: standard deviation | | | | | |

LITERATURE

N. Axelsen et al. in: A Manual of Quantitative Immunoelectrophoresis, Blackwell Scientific Publications, 1973, Chapter 23.

A. Johnstone and R. Thorpe, Immunochemistry in Practice, Blackwell Scientific Publications, 1982 (more specifically pp. 27-31).

Diderichsen, B., Wedsted, U., Hedegaard, L., Jensen, B. R., Sjoholm, C. (1990) Cloning of aldB, which encodes alpha-acetolactate decarboxylase, an exoenzyme from Bacillus brevis. J. Bacteriol., 172, 4315-4321.

Dretzen, G., Bellard, M., Sassone-Corsi, P., Chambon, P. (1981) A reliable method for the recovery of DNA fragments from agarose and acrylamide gels. Anal. Biochem., 112, 295-298.

Feinberg, A. P. and Vogelstein, B. (1983) Anal. Biochem. 132:6-13.

Maidak et al. (1996) The Ribosomal Database Project. Nucl. Acids Res. 24:82-85.

Zillig (1992) The order Thermococcales. In: The Procaryotes, Albert Barlows et al. (Ed.); Huber and Stetter (1992) The order Thermoproteales. In: The Procaryotes, Albert Barlows et al. (Ed.)

Halio et al. (1996) Sequence, expression in E. coli, and analysis of the gene encoding a novel intracellular protease (pfpi) from the hyperthermophilic archaeon Pyrococcus furiosus. J. Bacteriol. 178:2605-2612; Kengen et al. (1996) Sugar metabolism in hyperthermophiles. FEMS Microbiology Reviews 18:119-137.

Henrissat, B. 1991. A classification of glycosyl hydrolases based on amino acid sequence similaritites. Biochem. J., 280:309-316.

Henrissat, B., and A. Bairoch. 1993. New families in the classification of glycosyl hydrolases based on amino acid sequence similaritites. Biochem. J., 293:781-788.

Kengen (1993) Purification and characterization of an extremely thermostable beta-glucosidase from the hyperthermophilic archaeon P. furiosus. Eur. J. Biochem. 213:305-312.

Bauer et al. (1996) Comparison of a beta-glucosidase and a beta-mannosidase from the hyperthermophilic archaeon P. furiosus. J. Biol. Chem. 271:23749-23755.

Bragger et al. (1989) Very stable enzymes from extremely thermophilic archaebacteria and eubacteria. Appl. Environ. Microbiol. 31:556-561.

Vincken, J.P., Beldman, G., and Voragen, A.G.J. Substrate-specificity of endo-beta-1,4-glucanases - what determines xyloglucanase activity. Carbohydrate Research 298(4):299-310, 1997.

Sambrook et al. (1989) Molecular cloning: A laboratory manual, Cold Spring Harbor lab., Cold Spring Harbor, NY.

Ausubel, F. M. et al. (eds.) "Current protocols in Molecular Biology". John Wiley and Sons, 1995.

Harwood, C. R., and Cutting, S. M. (eds.) "Molecular Biological Methods for Bacillus". John Wiley and Sons, 1990.

O. Ouchterlony in: Handbook of Experimental Immunology (D.M. Weir, Ed.), Blackwell Scientific Publications, 1967, pp. 655-706.

Pitcher, D. G., Saunders, N. A., Owen, R. J. (1989). Rapid extraction of bacterial genomic DNA with guanidium thiocyanate. Lett. Appl. Microbiol. 8:151-156.

Eds. A.L. Sonenshein, J.A. Hoch and Richard Losick (1993) Bacillus subtilis and other Gram-Positive Bacteria, American Society for microbiology, p.618.

Yasbin, R.E., Wilson, G.A. and Young, F.E. (1975) Transformation and transfection in lysogenic strains of Bacillus subtilis : evidence for selective induction of prophage in competent cells. J. Bacteriol, 121:296-304.

McKenzie,T., Hoshino,T., Tanaka,T. and Sueoka,N. The nucleotide sequence of pUB110: some salient features in relation to replication and its regulation Plasmid 15 (2), 93-103 (1986)).

McKenzie,T., Hoshino,T., Tanaka,T. and Sueoka,N. Correction. A revision of the nucleotide sequence and functional map of pUB110 Plasmid 17 (1), 83-85 (1987).

Yansura et al.(1984) Use of E.coli lac repressor and operator to control gene expression in Bacillus subtilis, PNAS, Vol81, pp. 439-443.

**Claims**

1. A DNA construct comprising a DNA sequence encoding an enzyme having endo-β-1,4-glucanase activity and optimum activity at a temperature of at least 90°C, preferably at least 95°C, more preferably at least 100°C, which DNA sequence comprises

   a) the DNA sequence corresponding to the endo-β-1,4-glucanase encoding part of the DNA sequence obtainable from the plasmid in *Escherichia coli* DSM 11203, or
   b) an analogue of the DNA sequence corresponding to the endo-β-1,4-glucanase encoding part of the DNA sequence obtainable from the plasmid in *Escherichia coli* DSM 11203, which

   i) is at least 80% identical, with the DNA sequence corresponding to the endo-β-1,4-glucanase encoding part of the DNA sequence obtainable from the plasmid in *Escherichia coli* DSM 11203, or
   ii) encodes a polypeptide which is at least 50% identical, with the polypeptide of SEQ ID NO:2 or the polypeptide encoded by a DNA sequence comprising the DNA sequence corresponding to the endo-β-1,4-glucanase encoding part of the DNA sequence obtainable from the plasmid in *Escherichia coli* DSM 11203.

2. The DNA construct according to claim 1, in which the DNA sequence is isolated from or produced on the basis of a DNA library from a strain belonging to the genus *Pyrococcus,* even more preferably to the species *Pyrococcus furiosus,* especially *Pyrococcus furiosus,* DSM 3638.

3. The DNA construct according to any of the claims 1 or 2, in which the DNA sequence is isolated from *Escherichia coli,* DSM 11203.

4. The DNA construct according to any of the claims 1-3 which further comprises a DNA sequence encoding a cellulose binding domain (CBD).

5. The DNA construct according to claim 4 which further comprises a DNA sequence encoding a cellulose binding domain (CBD), the cellulose binding domain and the enzyme core (catalytically active domain) of the enzyme encoded by the DNA sequence of the DNA construct being operably linked.

6. A recombinant expression vector comprising a DNA construct according to any of claims 1-5.

7. A host cell into which a DNA construct according to any of claims 1-5 or a recombinant expression vector according to claim 6 has been introduced.

8. A cell according to claim 7, which is a prokaryotic cell, in particular a bacterial cell; or an eukaryotic cell, in particular a filamentous fungal cell; or an endogenous cell from which the DNA sequence, encoding an enzyme exhibiting endo-β-1,4-glucanase activity, originates.

9. A cell according to claim 8, wherein the bacterial cell belongs to a strain of *Bacillus,* preferably a strain of *Bacillus subtilis* or *Bacillus lentus;* and the fungal cell belongs to a strain selected from *Aspergillus ssp.* and *Fusarium ssp.*

10. A cell according to claim 7 or 8, wherein the cell belongs to a strain of *Pyrococcus,* preferably *Pyrococcus furiosus,* DSM 3638.

11. A cell according to claim 7 or 8, wherein the cell belongs to a strain of *Saccharomyces,* preferably a strain of *Saccharomyces cerevisiae.*

12. A method of producing an enzyme having endo-β-1,4-glucanase activity and optimum activity above 90°C, preferably above 95°C, more preferably above 100°C, the method comprising culturing a cell according to any of claims 7-11 under conditions permitting the production of the enzyme, and recovering the enzyme from the culture.

13. The method according to claim 12, wherein the recovered enzyme is at least 20% pure.

14. An isolated enzyme having endo-β-1,4-glucanase activity, in which the enzyme is (i) free from homologous impurities, and (ii) produced by the method according to claim 12 or 13.

15. An enzyme having endo-β-1,4-glucanase activity and optimum activity above 90°C, preferably above 95°C, more preferably above 100°C, which enzyme

    a) is encoded by a DNA construct according to any of claims 1-5, or
    b) is at least 60% identical with the amino acid sequence shown in SEQ ID NO:2, or
    c) is produced by the method according to claim 13, or
    d) is a polypeptide produced by *Pyrococcus furiosus,* DSM 3638, which is encodable by the endo-β-1,4-glucanase encoding part of the DNA sequence obtainable from the plasmid in *Escherichia coli* DSM 11203.

16. The enzyme according to claim 14 or 15, which belongs to family 12 of glycosyl hydrolases.

17. An enzyme preparation which is enriched in the enzyme according to any of the claims 14-16.

18. The preparation according to claim 17, which additionally comprises one or more enzymes selected from the group consisting of mannanases, galactanases, xylanases, arabinanases, pectin acetyl esterases, polygalacturonases, rhamnogalacturonases, pectin lyases, pectate lyases, pectin methylesterases, endo-β-1,4-glucanases, proteases, lipases, amylases, cutinases, peroxidases, laccases, cellobiohydrolases and transglutaminases.

19. An isolated substantially pure biological culture of the strain *Escherichia coli,* DSM 11203.

20. Use of the enzyme according to any of the claims 14-16 or the enzyme preparation according to claims 17 or 18 in the textile industry for improving the properties of cellulosic fibres or fabric or for providing a stone-washed look of denim; or in the cellulosic fiber processing industry; or in industrial cleaning processes; or in heat extruded polymeric material; or in the conversion of biomass to sugars; or in the production of alcohol; or for predigestion of e.g. grains used in the feed production; or in the production of instant coffee or similar extraction processes; or in the oil industry for degradation of aqueous solutions of hydrocolloid cellulose derivatives used in drilling or in enhanced oil recovery by polymer flooding.

**Patentansprüche**

1. DNA-Konstrukt umfassend eine DNA-Sequenz, kodierend ein Enzym mit endo-β-1,4-Glukanaseaktivität und optimaler Aktivität bei einer Temperatur von mindestens 90°C, vorzugsweise mindestens 95°C, stärker bevorzugt mindestens 100°C, welche DNA-Sequenz

    a) die DNA-Sequenz, entsprechend dem endo-β-1,4-Glukanase kodierenden Teil der DNA-Sequenz, erhältlich aus dem Plasmid in *Escherichia coli* DSM 11203, oder
    b) ein Analogon der DNA-Sequenz, entsprechend dem endo-β-1,4-Glukanase kodierenden Teil der DNA-Sequenz, erhältlich aus dem Plasmid in *Escherichia coli* DSM 11203, umfasst, welches

        i) mindestens 80% identisch zu der DNA-Sequenz ist, die dem endo-β-1,4-Glukanase kodierenden Teil der DNA-Sequenz entspricht, die aus dem Plasmid in *Escherichia coli* DSM 11203 erhältlich ist, oder
        ii) ein Polypeptid kodiert, welches mindestens 50% identisch zu dem Polypeptid von SEQ ID NO: 2 oder dem Polypeptid, das durch eine DNA-Sequenz kodiert ist, umfassend die DNA-Sequenz, die dem endo-β-1,4-Glukanase kodierenden Teil der DNA-Sequenz entspricht, die aus dem Plasmid in *Escherichia coli* DSM 11203 erhältlich ist, ist.

2. DNA-Konstrukt nach Anspruch 1, in welchem die DNA-Sequenz auf der Basis einer DNA-Bibliothek aus einem Stamm, der der Gattung *Pyrococcus,* noch stärker bevorzugt der Spezies *Pyrococcus furiosus,* insbesondere *Pyrococcus furiosus,* DSM 3638 angehört, isoliert oder hergestellt ist.

3. DNA-Konstrukt nach einem beliebigen der Ansprüche 1 oder 2, in welchem die DNA-Sequenz aus *Escherichia coli,* DSM 11203 isoliert ist.

4. DNA-Konstrukt nach einem beliebigen der Ansprüche 1-3, welches des Weiteren eine DNA-Sequenz umfasst, die eine Zellulose bindende Domäne (CBD) kodiert.

5. DNA-Konstrukt nach Anspruch 4, welches des Weiteren eine DNA-Sequenz umfasst, die eine Zellulose bindende Domäne (CBD) kodiert, wobei die Zellulose bindende Domäne und der Enzymkem (katalytisch aktive Domäne) des Enzyms, das durch die DNA-Sequenz des DNA-Konstrukts kodiert ist, funktionsfähig verknüpft sind.

6. Rekombinanter Expressionsvektor, umfassend ein DNA-Konstrukt gemäß einem beliebigen der Ansprüche 1-5.

7. Wirtszelle, in welche ein DNA-Konstrukt gemäß einem beliebigen der Ansprüche 1-5 oder ein rekombinanter Expressionsvektor gemäß Anspruch 6 eingeführt worden ist.

8. Zelle nach Anspruch 7, welche eine prokaryotische Zelle ist, insbesondere eine bakterielle Zelle, oder eine eukaryotische Zelle, insbesondere eine Zelle eines filamentösen Pilzes; oder eine endogene Zelle, aus welcher die DNA-Sequenz, die ein Enzym kodiert, das endo-β-1,4-Glukanaseaktivität aufweist, stammt.

9. Zelle nach Anspruch 8, wobei die bakterielle Zelle einem Bazillusstamm, vorzugsweise einem Stamm von *Bacillus subtilis* oder *Bacillus lentus* angehört; und wobei die Pilzzelle einem Stamm angehört, ausgewählt von *Aspergillus ssp.* und *Fusarium ssp.*

10. Zelle nach Anspruch 7 oder 8, wobei die Zelle einem Stamm von *Pyrococcus,* vorzugsweise *Pyrococcus furiosus,* DSM 3638, angehört.

11. Zelle nach Anspruch 7 oder 8, wobei die Zelle einem Stamm von *Saccharomyces,* vorzugsweise einem Stamm von *Saccharamyces cerevisiae* angehört.

12. Verfahren zum Herstellen eines Enzyms mit endo-β-1,4-Glukanaseaktivität und optimaler Aktivität oberhalb 90°C, vorzugsweise oberhalb 95°C, stärker bevorzugt oberhalb 100°C, wobei das Verfahren das Kultivieren einer Zelle gemäß einem beliebigen der Ansprüche 7-11 unter Bedingungen, die die Herstellung des Enzyms erlauben, und Gewinnen des Enzyms aus der Kultur, umfasst.

13. Verfahren nach Anspruch 12, wobei das gewonnene Enzym mindestens 20% rein ist.

14. Isoliertes Enzym mit endo-β-1,4-Glukanaseaktivität, in welchem das Enzym (i) frei von homologen Verunreinigungen ist, und (ii) durch das Verfahren gemäß Anspruch 12 oder 13 hergestellt ist.

15. Enzym mit endo-β-1,4-Glukanaseaktivität und optimaler Aktivität oberhalb 90°C, vorzugsweise oberhalb 95°C, stärker bevorzugt oberhalb 100°C, welches Enzym

    a) durch ein DNA-Konstrukt gemäß einem beliebigen der Ansprüche 1-5 kodiert ist, oder
    b) mindestens 60% identisch mit der in SEQ ID NO: 2 gezeigten Aminosäuresequenz ist, oder
    c) durch das Verfahren gemäß Anspruch 13 hergestellt ist, oder
    d) ein durch *Pyrococcus furiosus,* DSM 3638, hergestelltes Polypeptid ist, welches durch den endo-β-1,4-Glukanase kodierenden Teil der DNA-Sequenz kodierbar ist, das aus dem Plasmid in *Escherichia coli* DSM 11203 erhältlich ist.

16. Enzym nach Anspruch 14 oder 15, welches der Familie 12 der Glykosylhydrolasen angehört.

17. Enzymzubereitung, die bezogen auf das Enzym gemäß einem beliebigen der Ansprüche 14-16 angereichert ist.

18. Zubereitung nach Anspruch 17, welche zusätzlich ein oder mehrere Enzym(e) umfasst, ausgewählt aus der Gruppe bestehend aus Mannanasen, Galaktanasen, Xylanasen, Arabinanasen, Pektinacetylesterasen, Polygalakturonasen, Rhamnogalakturonasen, Pektinlyasen, Pektatlyasen, Pektinmethylesterasen, endo-β-1,4-Glukanasen, Proteasen, Lipasen, Amylasen, Cutinasen, Peroxidasen, Laccasen, Cellobiohydrolasen und Transglutaminasen.

19. Isolierte, im Wesentlichen reine biologische Kultur des Stamms *Escherichia coli,* DSM11203.

20. Verwendung des Enzyms gemäß einem beliebigen der Ansprüche 14-16 oder der Enzymzubereitung gemäß Ansprüchen 17 oder 18 in der Textilindustrie zum Verbessern der Eigenschaften von Zellulosefasern oder von Stoff oder zum Bereitstellen eines "stone-washed" Aussehen von Denim; oder in der Zellulosefasern verarbeitenden Industrie; oder in industriellen Reinigungsvorgängen; oder Hitze extrudierten polymeren Material; oder in der Umsetzung von Biomasse zu Zuckern; oder in der Herstellung von Alkohol; oder zur Vorverdauung von z.B. in der Futtermittelherstellung verwendeten Getreidekömern; oder in der Herstellung von Instantkaffee oder ähnlichen Extraktionsvorgängen; oder in der Ölindustrie zum Abbau von wässrigen Lösungen von hydrokolloidalen Zellulosederivaten, die beim Bohren oder in der verstärkten Ölrückgewinnung durch Polymerauffüllung verwendet werden.

**Revendications**

1. Produit de construction d'ADN comprenant une séquence d'ADN codant pour une enzyme ayant une activité endo-β-1,4-glucanase et une activité optimale à une température d'au moins 90 °C, de préférence au moins 95 °C, de manière davantage préférée au moins 100 °C, laquelle séquence d'ADN comprend

a) la séquence d'ADN correspondant à la partie codant pour l'endo-β-1,4-glucanase de la séquence d'ADN pouvant être obtenue à partir du plasmide dans *Escherichia coli* DSM 11203, ou
b) un analogue de la séquence d'ADN correspondant à la partie codant pour l'endo-β-1,4-glucanase de la séquence d'ADN pouvant être obtenue à partir du plasmide dans *Escherichia coli* DSM 11203, qui

i) est au moins identique à 80 %, avec la séquence d'ADN correspondant à la partie codant pour l'endo-β-1,4-glucanase de la séquence d'ADN pouvant être obtenue à partir du plasmide dans *Escherichia coli* DSM 11203, ou
ii) code pour un polypeptide qui est au moins identique à 50 %, avec le polypeptide de SEQ ID NO : 2 ou le polypeptide codé par une séquence d'ADN comprenant la séquence d'ADN correspondant à la partie codant pour l'endo-β-1,4-glucanase de la séquence d'ADN pouvant être obtenue à partir du plasmide dans *Escherichia coli* DSM 11203.

2. Produit de construction d'ADN selon la revendication 1, dans lequel la séquence d'ADN est isolée de ou produite sur la base d'une banque d'ADN provenant d'une souche appartenant au genre *Pyrococcus,* de manière même davantage préférée à l'espèce *Pyrococcus furiosus,* particulièrement *Pyrococcus furiosus,* DSM 3638.

3. Produit de construction d'ADN selon l'une quelconque des revendications 1 ou 2, dans lequel la séquence d'ADN est isolée de *Escherichia coli,* DSM 11203.

4. Produit de construction d'ADN selon l'une quelconque des revendications 1 à 3 qui comprend en outre une séquence d'ADN codant pour un domaine de liaison de la cellulose (CBD).

5. Produit de construction d'ADN selon la revendication 4, qui comprend en outre une séquence d'ADN codant pour un domaine de liaison de la cellulose (CBD), le domaine de liaison de la cellulose et le noyau de l'enzyme (domaine actif sur le plan catalytique) de l'enzyme codée par la séquence d'ADN du produit de construction d'ADN étant liée de manière fonctionnelle.

6. Vecteur d'expression recombinant comprenant un produit de construction d'ADN selon l'une quelconque des revendications 1 à 5.

7. Cellule hôte dans laquelle un produit de construction d'ADN selon l'une quelconque des revendications 1 à 5 ou un vecteur d'expression recombinant selon la revendication 6 a été introduit.

8. Cellule selon la revendication 7, qui est une cellule procaryote, en particulier une cellule bactérienne ; ou une cellule eucaryote, en particulier une cellule de champignon filamenteux ; ou une cellule endogène dont provient la séquence d'ADN codant pour une enzyme présentant une activité endo-β-1,4-glucanase.

9. Cellule selon la revendication 8, où la cellule bactérienne appartient à une souche de *Bacillus,* de préférence une souche de *Bacillus subtilis* ou *Bacillus lentus ;* et la cellule fongique appartient à une souche choisie parmi une sous-espèce de *Aspergillus* et une sous-espèce de *Fusarium.*

10. Cellule selon la revendication 7 ou 8, où la cellule appartient à une souche de *Pyrococcus,* de préférence *Pyrococcus furiosus,* DSM 3638.

11. Cellule selon la revendication 7 ou 8, où la cellule appartient à une souche de *Saccharomyces,* de préférence une souche de *Saccharomyces cerevisiae.*

**12.** Procédé de production d'une enzyme possédant une activité endo-β-1,4-glucanase et une activité optimale au-dessus de 90 °C, de préférence au-dessus de 95 °C, de manière davantage préférée au-dessus de 100 °C, le procédé comprenant la culture d'une cellule selon l'une quelconque des revendications 7 à 11 dans des conditions permettant la production de l'enzyme, et la récupération de l'enzyme à partir de la culture.

**13.** Procédé selon la revendication 12, dans lequel l'enzyme récupérée est au moins pure à 20 %.

**14.** Enzyme isolée possédant une activité endo-β-1,4-glucanase, où l'enzyme est (i) dépourvue d'impuretés homologues, et (ii) produite par le procédé selon la revendication 12 ou 13.

**15.** Enzyme possédant une activité endo-β-1,4-glucanase et une activité optimale au-dessus de 90 °C, de préférence au-dessus de 95 °C, de manière davantage préférée au-dessus de 100 °C, laquelle enzyme

a) est codée par une produit de construction d'ADN selon l'une quelconque des revendications 1 à 5, ou
b) est au moins identique à 60 % avec la séquence d'acides aminés représentée par SEQ ID NO : 2, ou
c) est produite par le procédé selon la revendication 13, ou
d) est un polypeptide produit par *Pyrococcus furiosus,* DSM 3638, qui peut être codé par la partie codant pour l'endo-β-1,4-glucanase de la séquence d'ADN pouvant être obtenue à partir du plasmide dans *Escherichia coli* DSM 11203.

**16.** Enzyme selon la revendication 14 ou 15, qui appartient à la famille 12 des glycosyl-hydrolases.

**17.** Préparation enzymatique qui est enrichie en l'enzyme selon l'une quelconque des revendications 14 à 16.

**18.** Préparation selon la revendication 17, qui comprend en outre une ou plusieurs enzymes choisies dans le groupe constitué de mannanases, galactanases, xylanases, arabinanases, pectine acétyl-estérases, polygalacturonases, rhamnogalacturonases, pectine lyases, pectate lyases, pectine méthyl-estérases, endo-β-1,4-glucanases, protéases, lipases, amylases, cutinases, peroxydases, laccases, cellobiohydrolases et transglutaminases.

**19.** Culture biologique substantiellement pure isolée de la souche de *Escherichia coli,* DSM 11203.

**20.** Utilisation de l'enzyme selon l'une quelconque des revendications 14 à 16 ou de la préparation enzymatique selon les revendications 17 ou 18 dans l'industrie du textile pour améliorer les propriétés des fibres ou du tissu cellulosiques ou pour améliorer un aspect « stone-washed » de denim ; ou dans l'industrie de traitement des fibres cellulosiques ; ou dans les procédés industriels de nettoyage ; ou dans le matériau polymère extrudé par la chaleur ; ou dans la conversion de biomasse en sucres ; ou dans la production d' alcool ; ou pour la prédigestion, par exemple, de graines utilisées dans la production d'aliments pour animaux ; ou dans la production de café instantané ou des procédés d'extraction similaires ; ou dans l'industrie pétrolière pour la dégradation de solutions aqueuses de dérivés de cellulose hydrocolloïde utilisées dans le forage ou dans l'amélioration de la récupération du pétrole par injection de polymère (« polymer flooding »).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DK 9000074 W
- WO 9744361 A
- GB 1368599 A
- EP 0307564 A
- EP 0435876 A
- WO 9117243 A
- WO 9110732 A
- WO 9117244 A
- DK 95000108 W
- DK 9500132 W
- WO 9000609 A
- WO 9516782 A
- WO 9419454 A
- US 5695976 A
- EP 0506780 B

### Non-patent literature cited in the description

- Microbial Cellulases. **T.-M. Enveri.** Microbial Enzymes and Biotechnology. Applied Science Publishers, 1983, 183-224
- Methods in Enzymology. 1988, vol. 160, 200-391
- **Béguin, P.** Molecular Biology of Cellulose Degradation. *Annu. Rev. Microbiol.,* 1990, vol. 44, 219-248
- **Béguin, P. ; Aubert, J-P.** The biological degradation of cellulose. *FEMS Microbiology Reviews,* 1994, vol. 13, 25-58
- **Henrissat, B.** Cellulases and their interaction with cellulose. *Cellulose,* 1994, vol. 1, 169-196
- **Henrissat, B. ; Bairoch, A.** *Biochem J.,* 1993, vol. 293, 781-788
- **Gilbert, H.J. ; Hazlewood, G.P.** *J. Gen. Microbiol.,* 1993, vol. 139, 187-194
- **Ford et al.** *Protein Expression and Purification,* 1991, vol. 2, 95-107
- **Cunningham ; Wells.** *Science,* 1989, vol. 244, 1081-1085
- **Vos et al.** *Science,* 1992, vol. 255, 306-312
- **Smith et al.** *J. Mol. Biol.,* 1992, vol. 224, 899-904
- **Wlodaver et al.** *FEBS Lett.,* 1992, vol. 309, 59-64
- Cellulose-Binding Domains: Classification and Properties. **Peter Tomme et al.** Enzymatic Degradation of Insoluble Carbohydrates. ACS Symposium Series, No. 618, 1996
- **Sambrook et al.** Molecular Cloning: A Laboratory Manual. Cold Spring Harbor Lab, 1989
- **Needleman, S.B. ; Wunsch, C.D.** *Journal of Molecular Biology,* 1970, vol. 48, 443-433
- **Needleman, S.B. ; Wunsch, C.D.** *Journal of Molecular Biology,* 1970, vol. 48, 443-453
- **Feinberg, A. P. ; Vogelstein, B.** *Anal. Biochem.,* 1983, vol. 132, 6-13
- **Lemesle-Varloot L ; Henrissat B ; Gaboriaud C ; Bissery V ; Morgat A ; Mornon Jp.** *Hydrophobic cluster analysis: Biochimie,* 1990, vol. 72 (8), 555-574
- **N. Axelsen et al.** A Manual of Quantitative Immunoelectrophoresis. Blackwell Scientific Publications, 1973
- **A. Johnstone ; R. Thorpe.** Immunochemistry in Practice. Blackwell Scientific Publications, 1982, 27-31
- **O. Ouchterlony.** Handbook of Experimental Immunology. Blackwell Scientific Publications, 1967, 655-706
- **Devereux et al.** *Nucleic Acids Res.,* 1984, vol. 12, 387-395
- **Lever, M.** A new reaction for colometric determination of carbohydrates. *Anal. Biochem.,* 1972, vol. 47, 273-279
- Current protocols in Molecular Biology. John Wiley and Sons, 1995
- **Diderichsen, B. ; Wedsted, U. ; Hedegaard, L. ; Jensen, B. R. ; Sjoholm, C.** Cloning of aldB, which encodes alpha-acetolactate decarboxylase, an exoenzyme from Bacillus brevis. *J. Bacteriol.,* 1990, vol. 172, 4315-4321
- **Dretzen, G. ; Bellard, M. ; Sassone-Corsi, P. ; Chambon, P.** A reliable method for the recovery of DNA fragments from agarose and acrylamide gels. *Anal. Biochem.,* 1981, vol. 112, 295-298
- **Maidak et al.** The Ribosomal Database Project. *Nucl. Acids Res.,* 1996, vol. 24, 82-85
- The order Thermococcales. **Zillig et al.** The Procaryotes. 1992
- The order Thermoproteales. **Huber ; Stetter et al.** The Procaryotes. 1992

- **Halio et al.** Sequence, expression in E. coli, and analysis of the gene encoding a novel intracellular protease (pfpi) from the hyperthermophilic archaeon Pyrococcus furiosus. *J. Bacteriol.,* 1996, vol. 178, 2605-2612
- **Kengen et al.** Sugar metabolism in hyperthermophiles. *FEMS Microbiology Reviews,* 1996, vol. 18, 119-137
- **Henrissat, B.** A classification of glycosyl hydrolases based on amino acid sequence similaritites. *Biochem. J.,* 1991, vol. 280, 309-316
- **Henrissat, B. ; A. Bairoch.** New families in the classification of glycosyl hydrolases based on amino acid sequence similaritites. *Biochem. J.,* 1993, vol. 293, 781-788
- **Kengen.** Purification and characterization of an extremely thermostable beta-glucosidase from the hyperthermophilic archaeon P. furiosus. *Eur. J. Biochem.,* 1993, vol. 213, 305-312
- **Bauer et al.** Comparison of a beta-glucosidase and a beta-mannosidase from the hyperthermophilic archaeon P. furiosus. *J. Biol. Chem.,* 1996, vol. 271, 23749-23755
- **Bragger et al.** Very stable enzymes from extremely thermophilic archaebacteria and eubacteria. *Appl. Environ. Microbiol.,* 1989, vol. 31, 556-561
- **Vincken, J.P. ; Beldman, G. ; Voragen, A.G.J.** Substrate-specificity of endo-beta-1,4-glucanases - what determines xyloglucanase activity. *Carbohydrate Research,* 1997, vol. 298 (4), 299-310
- **Sambrook et al.** Molecular cloning: A laboratory manual. Cold Spring Harbor lab, 1989
- Molecular Biological Methods for Bacillus. John Wiley and Sons, 1990
- **Pitcher, D. G. ; Saunders, N. A. ; Owen, R. J.** Rapid extraction of bacterial genomic DNA with guanidium thiocyanate. *Lett. Appl. Microbiol.,* 1989, vol. 8, 151-156
- Bacillus subtilis and other Gram-Positive Bacteria. American Society for microbiology, 1993, 618
- **Yasbin, R.E. ; Wilson, G.A. ; Young, F.E.** Transformation and transfection in lysogenic strains of Bacillus subtilis : evidence for selective induction of prophage in competent cells. *J. Bacteriol,* 1975, vol. 121, 296-304
- **McKenzie,T. ; Hoshino,T. ; Tanaka,T. ; Sueoka,N.** The nucleotide sequence of pUB110: some salient features in relation to replication and its regulation. *Plasmid,* 1986, vol. 15 (2), 93-103
- **McKenzie,T. ; Hoshino,T. ; Tanaka,T. ; Sueoka,N.** Correction. A revision of the nucleotide sequence and functional map of pUB110. *Plasmid,* 1987, vol. 17 (1), 83-85
- **Yansura et al.** Use of E.coli lac repressor and operator to control gene expression in Bacillus subtilis. *PNAS,* 1984, vol. 81, 439-443

SEQUENCE LISTING

(1) GENERAL INFORMATION:

    (i) APPLICANT:

        (A) NAME: NOVO NORDISK A/S
        (B) STREET: Novo Alle
        (C) CITY: Bagsvaerd
        (E) COUNTRY: Denmark
        (F) POSTAL CODE (ZIP): DK-2880
        (G) TELEPHONE: + 45 44 44 88 88
        (H) TELEFAX: +45 44 49 32 56

    (ii) TITLE OF INVENTION: A NOVEL ENDOGLUCANASE
    (iii) NUMBER OF SEQUENCES: 2
    (iv) COMPUTER READABLE FORM:

        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30 (EPO)

(2) INFORMATION FOR SEQ ID NO: 1:

    (i) SEQUENCE CHARACTERISTICS:

        (A) LENGTH: 960 base pairs
        (B) TYPE: nucleic acid
        (C) STRANDEDNESS: single
        (D) TOPOLOGY: linear

    (ii) MOLECULE TYPE: DNA (genomic)
    (xi) SEQUENCE DESCRIPTION: SEQ ID NO: 1:

```
ATGAGCAAGA AAAAGTTCGT CATCGTATCT ATCTTAACAA TCCTTTTAGT ACAGGCAATA        60

TATTTTGTAG AAAAGTATCA TACCTCTGAG GACAAGTCAA CTTCAAATAC CTCATCTACA       120

CCACCCCAAA CAACACTTTC CACTACCAAG GTTCTCAAGA TTAGATACCC TGATGACGGT       180

GAGTGGCCAG GAGCTCCTAT TGATAAGGAT GGTGATGGGA ACCCAGAATT CTACATTGAA       240

ATAAACCTAT GGAACATTCT TAATGCTACT GGATTTGCTG AGATGACGTA CAATTTAACC       300

AGCGGCGTCC TTCACTACGT CCAACAACTT GACAACATTG TCTTGAGGGA TAGAAGTAAT       360

TGGGTGCATG GATACCCCGA AATATTCTAT GGAAACAAGC CATGGAATGC AAACTACGCA       420

ACTGATGGCC CAATACCATT ACCCAGTAAA GTTTCAAACC TAACAGACTT CTATCTAACA       480

ATCTCCTATA AACTTGAGCC CAAGAACGGC CTGCCAATTA ACTTCGCAAT AGAATCCTGG       540

TTAACGAGAG AAGCTTGGAG AACAACAGGA ATTAACAGCG ATGAGCAAGA AGTAATGATA       600

TGGATTTACT ATGACGGATT ACAACCGGCT GGCTCCAAAG TTAAGGAGAT TGTAGTCCCA       660

ATAATAGTTA ACGGAACACC AGTAAATGCT ACATTTGAAG TATGGAAGGC AAACATTGGT       720

TGGGAGTATG TTGCATTTAG AATAAAGACC CCAATCAAAG AGGGAACAGT GACAATTCCA       780

TACGGAGCAT TTATAAGTGT TGCAGCCAAC ATTTCAAGCT TACCAAATTA CACAGAACTT       840


TACTTAGAGG ACGTGGAGAT TGGAACTGAG TTTGGAACGC CAAGCACTAC CTCCGCCCAC       900

CTAGAGTGGT GGATCACAAA CATAACACTA ACTCCTCTAG ATAGACCTCT TATTTCCTAA       960
```

(2) INFORMATION FOR SEQ ID NO: 2:

    (i) SEQUENCE CHARACTERISTICS:

        (A) LENGTH: 319 amino acids
        (B) TYPE: amino acid
        (C) STRANDEDNESS:
        (D) TOPOLOGY: linear

    (ii) MOLECULE TYPE: protein
    (xi) SEQUENCE DESCRIPTION: SEQ ID NO: 2:

```
Met Ser Lys Lys Lys Phe Val Ile Val Ser Ile Leu Thr Ile Leu Leu
1               5                   10                  15

Val Gln Ala Ile Tyr Phe Val Glu Lys Tyr His Thr Ser Glu Asp Lys
            20              25                  30

Ser Thr Ser Asn Thr Ser Ser Thr Pro Pro Gln Thr Thr Leu Ser Thr
        35              40                  45

Thr Lys Val Leu Lys Ile Arg Tyr Pro Asp Asp Gly Glu Trp Pro Gly
    50              55                  60

Ala Pro Ile Asp Lys Asp Gly Asp Gly Asn Pro Glu Phe Tyr Ile Glu
65              70                  75                      80

Ile Asn Leu Trp Asn Ile Leu Asn Ala Thr Gly Phe Ala Glu Met Thr
            85                  90                      95

Tyr Asn Leu Thr Ser Gly Val Leu His Tyr Val Gln Gln Leu Asp Asn
            100             105                 110

Ile Val Leu Arg Asp Arg Ser Asn Trp Val His Gly Tyr Pro Glu Ile
        115                 120                 125

Phe Tyr Gly Asn Lys Pro Trp Asn Ala Asn Tyr Ala Thr Asp Gly Pro
    130             135                 140

Ile Pro Leu Pro Ser Lys Val Ser Asn Leu Thr Asp Phe Tyr Leu Thr
145             150                 155                 160

Ile Ser Tyr Lys Leu Glu Pro Lys Asn Gly Leu Pro Ile Asn Phe Ala
            165                 170                 175

Ile Glu Ser Trp Leu Thr Arg Glu Ala Trp Arg Thr Thr Gly Ile Asn
            180             185                 190

Ser Asp Glu Gln Glu Val Met Ile Trp Ile Tyr Tyr Asp Gly Leu Gln
        195                 200                 205

Pro Ala Gly Ser Lys Val Lys Glu Ile Val Val Pro Ile Ile Val Asn
    210                 215                 220

Gly Thr Pro Val Asn Ala Thr Phe Glu Val Trp Lys Ala Asn Ile Gly
225                 230                 235                     240

Trp Glu Tyr Val Ala Phe Arg Ile Lys Thr Pro Ile Lys Glu Gly Thr
            245                 250                 255

Val Thr Ile Pro Tyr Gly Ala Phe Ile Ser Val Ala Ala Asn Ile Ser
            260             265                 270

Ser Leu Pro Asn Tyr Thr Glu Leu Tyr Leu Glu Asp Val Glu Ile Gly
    275             280                 285

Thr Glu Phe Gly Thr Pro Ser Thr Thr Ser Ala His Leu Glu Trp Trp
    290             295                 300

Ile Thr Asn Ile Thr Leu Thr Pro Leu Asp Arg Pro Leu Ile Ser
305             310                 315
```

22